(19) **Europäisches Patentamt / European Patent Office / Office européen des brevets**

(11) **EP 1 796 831 B1**

(12) **EUROPEAN PATENT SPECIFICATION**

(45) Date of publication and mention of the grant of the patent:
**15.01.2020 Bulletin 2020/03**

(51) Int Cl.:
*B01J 20/26* (2006.01)  *B01J 20/30* (2006.01)
*A61L 15/60* (2006.01)  *A61F 13/15* (2006.01)
*A61F 13/53* (2006.01)

(21) Application number: **05780289.4**

(22) Date of filing: **05.08.2005**

(86) International application number:
**PCT/JP2005/014809**

(87) International publication number:
**WO 2006/014031 (09.02.2006 Gazette 2006/06)**

(54) **METHOD FOR PRODUCTION OF A PARTICULATE WATER-ABSORBING AGENT, CORRESPONDING PARTICULATE WATER-ABSORBING AGENT, AND ABSORBING ARTICLE**

VERFAHREN ZUR HERSTELLUNG EINES TEILCHENFÖRMIGEN WASSERABSORBIERENDEN MITTELS, ENTSPRECHENDES TEILCHENFÖRMIGES WASSERABSORBIERENDES MITTEL UND ABSORBIERENDER ARTIKEL

PROCÉDE DÉ PRODUCTION D'UN AGENT PARTICULAIRE ABSORBANT L'EAU, AGENT PARTICULAIRE ABSORBANT L'EAU CORRESPONDANT ET ARTICLE ABSORBANT

(84) Designated Contracting States:
**DE FR**

(30) Priority: **06.08.2004 JP 2004230574**

(43) Date of publication of application:
**20.06.2007 Bulletin 2007/25**

(73) Proprietor: **Nippon Shokubai Co.,Ltd.**
**Osaka-shi, Osaka 541-0043 (JP)**

(72) Inventors:
• **WADA, Katsuyuki**
**Hyogo 670-0081 (JP)**
• **UEDA, Hiroko**
**Hyogo 671-1235 (JP)**
• **KANTO, Teruyuki**
**Hyogo 671-1235 (JP)**

• **ISHIZAKI, Kunihiko**
**Osaka 565-0875 (JP)**
• **IMURA, Motohiro**
**Hyoko 673-0891 (JP)**

(74) Representative: **Glawe, Delfs, Moll**
**Partnerschaft mbB von**
**Patent- und Rechtsanwälten**
**Postfach 13 03 91**
**20103 Hamburg (DE)**

(56) References cited:
WO-A1-03/095510     JP-A- 11 156 188
JP-A- 2001 098 170     JP-A- 2002 121 291
JP-A- 2004 156 010     US-A- 4 732 968
US-A1- 2004 048 955

**Description**

Technical Field

The present invention relates to a particulate water-absorbing agent including a water-absorbing resin as a main component. In particular, it relates to a particulate water-absorbing agent fulfilling more superior absorption ability without generating odor in practical applications as absorbing articles such as a disposable diaper(hereinafter referred to as a diaper).

Background Art

At present, as component materials in sanitary goods such as a disposable diaper, a sanitary napkin, an incontinence pad, and the like, a water-absorbing resin (water-absorbing agent) to absorb body fluid and hydrophilic fibers such as pulp are widely used. As the water-absorbing resin, for example, partially neutralized and crosslinked polyacrylic acid, hydrolysates of starch-acrylic acid graft polymer, saponified vinyl acetate-acrylic acid ester copolymers, hydrolysates of acrylonitrile copolymers or acrylamide copolymers, or crosslinked polymers thereof, crosslinked polymers of cationic monomers, and the like are used as main raw materials .

Water-absorption properties suggested to be desired for the water-absorbing resin have conventionally included high absorption capacity, excellent absorption speed, liquid permeability and gel strength of swollen gel in contact with aqueous liquid such as bodily fluid, as well as absorbing amount for sucking water from a substrate containing aqueous liquid. Accordingly, water-absorbing agents adjusted to have specific property have been proposed.

Such a water-absorbing resin has been required to have superior property such as liquid absorption amount, water absorption speed, gel strength and gel permeability in contact with aqueous liquid such as body fluid, along with superior water suction force to suck water from a substrate containing aqueous liquid. Further, as recent trends, water-absorbing resin powder has been expected to exhibit very narrow particle size distribution, with high absorption capacity and low water-extractables. In addition, high absorbency against pressure or liquid permeability under pressure has essentially been required.

For example, there are many patent applications on many parameters specifying various properties of these water-absorbing resins, or on measurement methods thereof (US Reissued 32649, UK2267094B, US5051259, US5419956, US6087002, EP0629441, EP0707603, EP0712659, EP1029886, US5462972, US5453323, US5797893, US6127454, US6184433, US6297335, US Reissued 37021, US5140076, US6414214B1, US5994440, US6444744, US6194531, EP0940148, EP1153656, EP0605215, US5147343, US5149335, EP0532002, US5601452, US5562646, US5669894, US6150582, WO02/053198, EP0937739, JP60158861, JP11241030, US4732968, WO03/095510 and US6388000).

Water-absorbing resins superior in gel strength, extractables and absorption capacity are proposed in US Reissued 32649. A water-absorbing resin superior in liquid permeability under no pressure, water absorption speed and absorption capacity is proposed in UK2267094B. Technology specifying specific particle size distribution is proposed in US5051259, US5419956, US6087002 and EP0629441. Further, a water-absorbing resin superior in absorbency against pressure under various loads, or many measurement methods therefore are also proposed, and water-absorbing resins with superior absorbency against pressure alone or in combination with other property are proposed in EP0707603, EP0712659, EP1029886, US5462972, US5453323, US5797893, US6127454, US6184433, US6297335, US Reissued 37021 and the like.

Moreover, water-absorbing resins with superior resistance to impact that decreases the property are proposed inUS5140076, US6414214B1 and the like. A water-absorbing resin with specific powdery dust amount is proposed in US5994440 and the like, and a water-absorbing resin with less coloring is proposed in US6444744 and the like. Water-absorbing resins superior in gel durability in an aqueous L-ascorbic acid solution as index of urine resistance or superior in water absorption ability are proposed in US6194531 and EP0940148. A water-absorbing resin with superior air permeability is proposed in EP1153656. A water-absorbing resin with lower amount of residual monomers is proposed in EP0605215.

Further, in US5147343, US5149335, EP0532002, US5601452, US5562646, US5669894, US6150582, WO02/053198 and the like, water-absorbing resins with specific property are proposed as suitable to absorbing articles such as a diaper having specific property, composition or polymer concentration.

Furthermore, hygiene materials such as a disposable diaper have changed into thinner type, in other words, the water-absorbing resin concentration tends to be increased in recent years, and thus, demands for improvement in connection with odors generated from a water-absorbing resin have increased.

With respect to odors, in attempts to impart deodorization performance to a water-absorbing resin, combinations of a water-absorbing resin and various types of deodorants or antimicrobials have been hitherto proposed. For example, a water-absorbing resin composition comprising a water-absorbing resin and an extract from leaves of Theaceae plant (for example, see, JP60158861), and a water-absorbing resin composition comprising an extract from conifer plant and a water-absorbing resin having a specific performance (for example, see, JP11241030) and the like have been known. A technique of agglomerating a water-absorbing resin in a dispersion solvent for particle size control has been known (US4732968). Also, in recent years, a technique focused on acetic acid and propionic acid which are impurities in acrylic acid as the odor of water-absorbing resins was proposed (WO03/095510). Additionally, in connection with residual monomer, a technique focused on β-hydroxy propionic acid which is an impurity in acrylic acid salt was also proposed (US6388000).

Many techniques have been proposed as described above, however, using amount of the water-absorbing resin has increased in absorbing cores of disposable diapers and the like in recent years, therefore, the absorbing cores are trended to have high water-absorbing resin concentrations (weight ratio of water-absorbing resin being high). Accordingly, there was a problem that conventional water-absorbing resins have not exhibited sufficient performance for use at high concentrations, and that also deodorization performance is not sufficient at high concentrations. As using amount of water-absorbing resin has increased, reduction of the amount of residual monomer has been further required.

An object of the present invention is to provide a particulate water-absorbing agent comprising a water-absorbing resin which is suited for practical applications at high concentrations in absorbing cores such as a diaper to supply superior absorbing articles, and a method for production of the same. More specifically, as means for solving problems (supplying superior absorbing articles), the present invention provides a water-absorbing agent which has an additional function and the water-absorbing agent being superior in deodorization performance with no odor generated after swelling, and being suited for practical applications, and a method for production of the same.

Disclosure of the Invention

Conventionally, attentions have been focused on an odor of urine in investigations of deodorization performances of a water-absorbing resin. The present inventors found in the process of elaborate investigations of deodorization performances of a water-absorbing resin as an additional function, that water-absorbing resins, particularly water-absorbing resins obtained by reversed phase suspension polymerization, themselves have a specific odor, and that the odor of the water-absorbing resin itself deteriorates the deodorization performance in practical applications. In addition, as an important factor for practical applications in a diaper, it was found that specific narrow particle size distribution and specific absorption capacity of a water-absorbing resin and the particle shape are of importance.

It was conventionally believed that water-absorbing resins produced by reversed phase suspension polymerization include less amount of residual monomer, however, as a result of elaborate investigations on cause for a specific odor of the water-absorbing resin itself produced by reversed phase suspension polymerization, it was found that the residual monomer is generated and the amount thereof is increased in fact. In addition, as a result of exploration of cause for the odor, increase of the amount of residual monomer, volatile organic solvent used in the reversed phase suspension polymerization, and generation of an odor derived from other impurities in monomer in reversed phase suspension polymerization were found.

After elaborate investigations to solve the problems, it was found that because polymerization, surface crosslinking, and drying and agglomeration were carried out in an organic solvent having low boiling points (for example, cyclohexane having a boiling point of 80.7°C) for the water-absorbing agents produced by conventional reversed phase suspension polymerization, a slight amount of the organic solvent remains according to conventional methods for production and in conventional products, and this organic solvent accounts for the odor in practical applications of the diaper. According to an aspect of the present invention, the problems are resolved for reducing the odor, by controlling minor components (acetic acid, propionic acid, acrylic acid dimer and the like) in the monomer, particularly in acrylic acid, without substantially using an organic solvent in surface crosslinking after reversed phase suspension polymerization, and by heating at high temperature (preferably not lower than 150°C and not higher than 250°C) also without substantially using an organic solvent in agglomeration.

Therefore, a method for production of a particulate water-absorbing agent according to an aspect of the present invention is a method as defined in claim 1.

A particulate water-absorbing agent according to the invention is defined in claim 2.

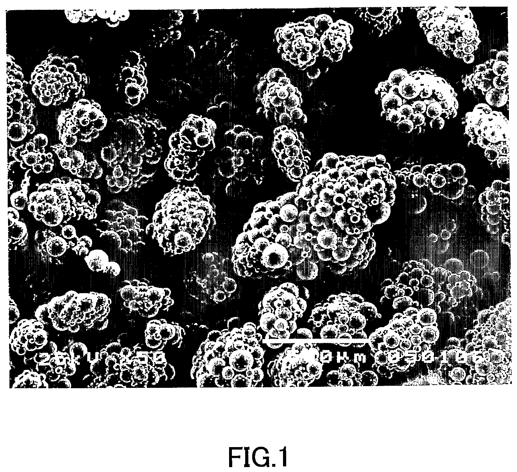

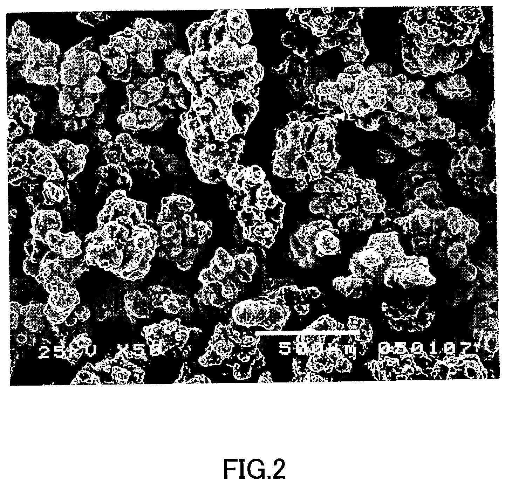

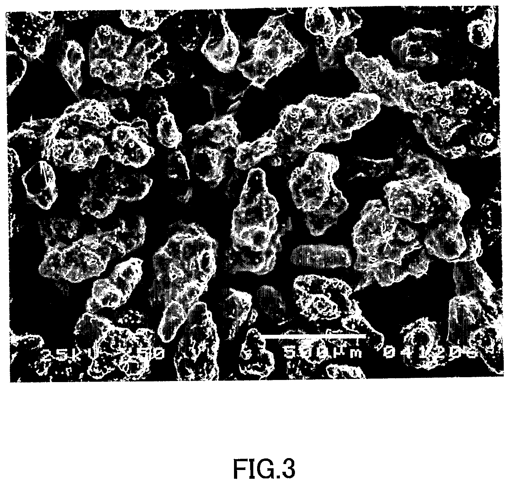

A nearly spherical shape referred to in this desription herein means a spherical or elliptical particule (including perfect spherical particulate shape) without having any angulate corner and having a ratio of the average major axis and average minor axis of the particle being not lower than 1.0 and not higher than 3.0, with the upper limit being preferably 2.0, and more preferably 1.5. The aggregate of the nearly spherical shape referred to herein means particles formed by aggregation of nearly spherical shapes which are primary particles to give a shape like bunch of grapes. Specific examples are illustrated in the electron micrograph shown in Fig. 1 described later. The aggregate derived from nearly spherical shapes referred to herein means rock-shaped particles, rice grain-shaped particles and the like formed by aggregation, fusion and integration of the nearly spherical shapes which are primary particles. Specific examples are illustrated in electron micrograph shown in Fig. 2 and Fig. 3 described later. In these cases, presence of the nearly spherical shapes which are primary particles can not be identified in appearance. As particle shape of a water-absorbing resin obtained by reversed phase suspension polymerization,a shape of particles with wrinkles is illustrated in Fig.1~Fig.4 of EP516925B1 (WO92/16565,US5744564). A shape of particles with perfect spherical body is illustrated in Fig.5 of EP516925B1. A sausage shape of particles is illustrated in Fig.2 of US4973632. The water-absorbing resin particles of the present invention are at least one kind or not less than 2 kinds of nearly spherical shapes, aggregates thereof and aggregates derived from nearly spherical shapes, and may be selected from them.

The particulate water-absorbing agent referred to herein means an absorbing and solidifying of an aqueous liquid, which comprises a water-absorbing resin as a main component, and contains a small amount of additive or water as needed. Content of the water-absorbing resin is not lower than 70% by weight and not higher than 100% by weight of the entire water-absorbing agent, and the lower limit thereof is preferably 80% by weight, and more preferably 90% by weight. In the minor component, water is generally a main component or essential, and in addition thereto, additives described later may be used. The aqueous liquid is not limited to water, but may be any one as long as it contains water such as e.g., urine, blood, excrement, waste liquid, humidity or steam, ice,a mixture of water and organic solvents or inorganic solvents, rain water and underground water and the like but not particularly limited thereto. Preferably, the absorbing and solidifying may be used for urine, and particularly human urine.

Also, measurement method of the content of volatile organic compounds as atmospheric concentration measured by a gas detector tube in the above item (d) is according to the measurement method (8) described in Examples below.

A water-absorbing agent (or a water-absorbing resin) of6.00g is added to the sniffing bag, and odorless air of 1.2 L is fed in the sniffing bag. Subsequently, 30 ml of a 0.90% by weight aqueous solution of sodium chloride is poured to the water-absorbing agent in the sniffing bag. The water-absorbing agent is left to permit swelling. The sniffing bag is left to stand in an incubator at 37°C for 60 minutes, and subsequently, left to stand at room temperature for 10 minutes. Then, atmospheric concentration of volatile organic compounds is measured using a gas detector tube. In the present invention, the content of volatile organic compounds is not a content of volatile organic compounds in a water-absorbing agent. The content of volatile organic compounds is an amount of gas in the atmosphere which is specified by a measuring method mentioned later. The inventors found that the content of volatile organic compounds specified in the present invention correlates to odor by a water-absorbing articles in practical applications (i.e. as a diaper, especially as a high concentration diaper), and completed the present invention.

According to the water-absorbing agent of the present invention, unprecedentedly comfortable use is enabled without generation of odor after swelling, and excellent absorption ability is achieved upon practical applications in absorbing articles such as a diaper.

Brief Description of the Drawings

Fig. 1 illustrates an electron micrograph showing a water-absorbing resin forming particles with a shape like bunch of grapes through aggregation of nearly spherical shapes which are primary particles.
Fig. 2 illustrates an electron micrograph showing a water-absorbing resin forming rock-shaped particles through aggregation and fusion of nearly spherical shapes which are primary particles.
Fig. 3 illustrates an electron micrograph showing a water-absorbing resin forming rice grain-shaped particles through aggregation and fusion of nearly spherical shapes which are primary particles.

Best Mode for Carrying Out the Invention

Raw materials used for the water-absorbing resin and the water-absorbing agent of the present invention and reaction conditions will be explained below.

(1) A water-absorbing resin

A water-absorbing resin of the present invention means a cross-linked polymer which can form hydrogel and is water swelling and non-dissolving in water. For example, water swelling indicates one absorbing large quantity of water in ion exchanged water, such as essentially 5 times or more own weight and preferably 50 to 1000 times, while non-dissolving in water means one with extractables of not higher than 50% by weight (lower limit:0% by weight) and still more, in the range described later. Measurement methods thereof are specified in Examples.

As the water-absorbing resin in the present invention, to attain objectives of the present invention, a water-absorbing resin obtained by crosslinking polymerization of an unsaturated monomer containing an acid group and/or salts thereof (may be a water-absorbing resin having a cross-linked and polymerized structure, and may also be a water-absorbing resin obtained by a crosslinking reaction with a crosslinking agent following polymerization of an unsaturated monomer containing an acid group and/or a salt thereof) is used, wherein partially neutralized polymer of polyacrylic acid obtained by polymerizing and crosslinking of an unsaturated monomer mainly composed of acrylic acid and/or salts (neutralized product) thereof is used.

(2) An unsaturated monomer

As an unsaturated monomer composing the water-absorbing resin (hereinafter, abbreviated simply as a monomer), acrylic acid and/or a salt thereof is used as a main component. Further, in order to obtain the water-absorbing agent of the present invention, acrylic acid is provided having a content of acetic acid and propionic acid in acrylic acid being not higher than 500 ppm (lower limit being 0 ppm), and acrylic acid dimer content being not higher than 1000 ppm (lower limit being 0 ppm). The content of acetic acid and propionic acid in acrylic acid, and the acrylic acid dimer content are not higher than 500 ppm, more preferably not higher than 300 ppm, and particularly preferably not higher than 100 ppm, respectively. The content of acetic acid and propionic acid being higher than 500 ppm in acrylic acid, and the acrylic acid dimer content being higher than 1000 ppmmay result in generation of the odor or the residual monomer, therefore, the water-absorbing agent of the present invention with less amount of volatile organic compounds can not be obtained.

In a method for obtaining the acrylic acid described above, oxidizing conditions for preparing acrylic acid may be controlled, or conditions for distillation and crystallization may be strictly controlled to fall within the above range. Degree of rectification may be respectively elevated by increase in number of theoretical plates (for example, increase 6 to 20 plates more than before), increase in reflux ratio and the like when a distillation method is employed as a purification method, or alternatively, by increase in number of times of crystallization (for example, increase 3 to 10 times) when a crystallization method is employed as a purification method. The distillation method and the crystallization method may be also employed in combination. Moreover, during storage of the acrylic acid following purification, the temperature may be controlled at a low temperature (preferably10 to 25°C,more preferably14 to 23°C).

In addition, other monomer except for acrylic acid may be used in combination. Examples of the monomer which may be additionally used include aqueous or hydrophobic unsaturated monomers such as methacrylic acid, maleic anhydride, maleic acid, fumaric acid, crotonic acid, itaconic acid, vinylsulfonic acid, 2-(meth)acrylamide-2-methylpropane sulfonic acid, (meth)acryloxyalkane sulfonic acid and its alkali metal salts, ammonium salts, N-vinyl-2-pyrrolidone, N-vinylacetamide, (meth)acrylamide, N-isopropyl(meth)acrylamide, N,N-dimethyl(meth)acrylamide, 2-hydroxyethyl (meth)acrylate, methoxypolyethyleneglycol (meth)acrylate, polyethyleneglycol (meth)acrylate, isobutylene, lauryl (meth)acrylate, and the like. These can be a copolymerization component.

When monomers other than acrylic acid (salts) are used in combination, to attain objectives of the present invention, use ratio of the monomer (excluding the following a crosslinkable monomer) other than acrylic acid (salt thereof) is preferably 0 to 30% by mole based on total amount with acrylic acid and salts thereof used as a main component, more preferably 0 to 10% by mole and most preferably 0 to 5% by mole.

When an unsaturated monomer containing an acid group is used as a monomer, examples of the salt thereof include alkali metal salts, alkaline earth metal salts and ammonium salts. They may be used in combination.In view of performance, industrial availability and safety of the water-absorbing resin obtained, sodium salts or potassium salts are preferable.

Also, an unsaturated monomer containing an acid group such as acrylic acid is preferably neutralized at the acid group in view of property and pH. Neutralization ratio of the acid group is usually 20 to 100% by mole, preferably 30 to 95% by mole, and more preferably 40 to 80% by mole. Neutralization of the acid group may be conducted with a monomer or with a polymer, either of which may be used in combination.

(3) A crosslinkable monomer (an internal crosslinking agent)

The water-absorbing resin according to the present invention is a cross-linked polymer and the crosslinkage may be a self-crosslinked type without using a crosslinkable monomer. However, in view of property, it is preferably obtained by copolymerization or reaction of th the above-mentioned monomer or polymer ,with a crosslinkable monomer (may be also referred to as an internal crosslinking agent of the water-absorbing resin) having not less than 2 polymerizable unsaturated groups or not less than 2 reactable groups in a molecule. Being a cross-linked polymer may be also specified by being non-dissolving in water as described above.

Specific examples of these internal crosslinking agent include, for example, N,N'-methylenebis(meth)acrylamide, (poly)ethyleneglycol di(meth)acrylate, (poly)propyleneglycol di(meth)acrylate, trimethylolpropane tri(meth)acrylate, glycerine tri(meth)acrylate, glycerine acrylate methacrylate, ethylene oxide modified trimethylolpropane tri(meth)acrylate, pentaerythritol tetra(meth)acrylate, triallyl cyanurate, triallyl isocyanurate, triallyl phosphate, triallylamine, poly(meth)allyloxyalkane, (poly)ethyleneglycol diglycidyl ether, glycerol diglycidyl ether, ethyleneglycol, polyethyleneglycol, propyleneglycol, glycerine, pentaerythritol, ethylenediamine, ethylene carbonate, propylene carbonate, polyethyleneimine, glycidyl (meth)acrylate, and the like.

These internal crosslinking agents may be used alone or in a mixture of two or more kinds, as appropriate. These internal crosslinking agents may be added as a whole into a reaction system or in portion wise. When at least one kind or not less than 2 kinds of internal crosslinking agents are used, in consideration of absorption properties of the water-absorbing resin or the water-absorbing agent finally obtained, it is preferable to use essentially a compound having not less than two polymerizable unsaturated groups, in polymerization.

Using amount of these internal crosslinking agents is preferably in the range of 0.001 to 2% by mole based on the aforementioned monomer (excluding the internal crosslinking agents), more preferably 0.005 to 0.5% by mole, further preferably 0.01 to 0.2% by mole, and particularly preferably 0.03 to 0.15% by mole. The using amounts of the internal crosslinking agents less than 0.001% by mole and over 2% by mole may not provide sufficient absorption properties.

When crosslinked structure is introduced inside a polymer by using the internal crosslinking agent, the internal crosslinking agent may be added to a reaction system before, during or after polymerization of the monomer, or after neutralization.

(4) A polymerization solvent

In the present invention, in view of property, the monomer is dissolved in polymerization solvent (especially water), and the monomer in the solution is polymerized by reversed phase suspension polymerization.
Monomer concentration in the aqueous solution(hereinafter referred to as a monomer aqueous solution),when an aqueous solution of the monomer is prepared,is determined by temperature of the aqueous solution or by the types of the monomer and not especially limited,however,preferably 10 to 70% by weight, and further preferably 20 to 60% by weight.When aqueous solution polymerization iscarried out, a solvent other than water may also be used, if necessary, a solvent type used in combination is not especially limited.

(5) A polymerization initiator

An initiator used in polymerization of the monomer to obtain the water-absorbing resin used in the present invention includes a radical polymerization initiator such as potassium persulfate, ammonium persulfate, sodium persulfate, potassium peracetate, sodium peracetate, potassium percarbonate, sodium percarbonate, tert-butyl hydroperoxide, hydrogen peroxide or 2,2'-azobis(2-amidinopropane) dihydrochloride; or a photopolymerization initiator such as 2-hydroxy-2-methyl -1-phenylpropane-1-one. They may be used for polymerization initiation by redox or ultraviolet rays. Using amount of the polymerization initiator is, in view of property, 0.001 to 2% by mole, preferably 0.01 to 0.1% by mole (based on total monomers). When the using amount of the polymerization initiator is less than 0.001% by mole, unreacted residual monomers increase, while, when the amount of the polymerization initiator is over 2% by mole, polymerization control becomes difficult and thus not preferable.

(6) A polymerization method

When the monomer is polymerized in order to obtain the water-absorbing agent of the present invention, reversed phase suspension polymerization is employed in which an aqueous solution of this monomer is dispersed in a hydrophobic organic solvent that is inert to polymerization in the presence of a dispersant to allow for polymerization. This polymerization method is described in, for example, US4093776, US4367323, US4446261, US4683274, US5185413,US5244735, US5998553, US6573330 and the like. Monomers, crosslinking agents, solvents, initiators and the like exemplified in these polymerization methods may be also employed in the present invention.

The reversed phase suspension polymerization is a polymerization method in which an aqueous solution of a polymerizable monomer is suspended or emulsified in a hydrophobic organic solvent to allow the monomer to be dispersed. Illustrative examples of the surfactant (US6458896) or dispersant for allowing the monomer to be dispersed include e.g., anionic surfactants, nonionic surfactants(US2003-153887), cationic surfactants, amphoteric surfactants and the like. Usable surfactant is exemplified in, for examle, US6107358.

Specific examples of the anionic surfactant which may be used include fatty acid sodium such as mixed fatty acid sodium soap and sodium stearate; higher alcohol sodium sulfate, sodium alkylsulfate, alkyl benzene sulfonate and the like. Examples of the nonionic surfactant include polyoxyethylene alkyl ether such as polyoxyethylene higher alcohol ether, sorbitan fatty acid esters, glycerol fatty acid esters and the like. Examples of the cationic surfactant and amphoteric surfactant include alkylamines, alkylbetaine and the like. Additionally, examples of other dispersant include ethyl cellulose, hydroxyethyl cellulose and the like.

Using amount of these surfactants or dispersants may be selected as appropriate depending on kind of the polymerization. In general, the using amount is preferably 0.1 to 30 parts by weight, and more preferably 0.3 to 5 parts by weight based on 100 parts by weight of entire monomer components including the polymerizable monomer and the crosslinkable monomer. Also, using amount of these dispersants or surfactants may be 0.001 to 10% by weight, and preferably 0.001 to 1% by weight based on the organic solvent described later.

The organic solvent (dispersion solvent) for use in the reversed phase suspension polymerization is hydrocarbons selected from n-hexane, n-heptane and cyclohexane in view of stability of industrial obtention , quality and the like. Using amount of these hydrophobic solvents may be 0.5 to 10 parts by weight, and preferably 0.6 to 5 parts by weight based on 1 part by weight of the aqueous solution containing the polymerizable monomer.

(7) Addition of a chain transfer agent

In the present invention, a chain transfer agent may be used in polymerization. By polymerization in the presence of an aqueous chain transfer agent in addition to the unsaturated monomer, internal crosslinking agent and polymerization initiator, and when the water-absorbing resin thus obtained is used as the water-absorbing agent of the present invention, an absorbing core with high absorbing ability and superior stability to urine can be obtained.

The aqueous chain transfer agent used for polymerization in the present invention is not especially limited as long as it dissolves in water or an aqueous ethylenic unsaturated monomer, and examples of the agent include thiols, thiolates, secondary alcohols, amines, hypophosphites and the like. Specific examples include mercaptoethanol, mercaptopropanol, dodecylmercaptan, thioglycols, thiomalic acid, 3-mercaptopropionic acid, isopropanol, sodium hypophosphite, formic acid and salts thereof. Although one kind or not less than 2 kinds selected from the group can be used, in view of the effect, a hypophosphite such as sodium hypophosphite is preferably used. Using amount of the aqueous chain transfer agent depends on kind of the aqueous chain transfer agent and concentration of a monomer aqueous solution, however, is 0.001 to 1% by mole, and preferably 0.005 to 0.3% by mole based on total monomers.

(8) Drying

After conducting reversed phase suspension polymerization according to the method of the present invention, depending on the water content following the polymerization, the resulting hydrated gel-like polymer is further dried to put into use as the water-absorbing resin. The drying method is not particularly limited but any known drying method can be used, for example, a variety of method such as a method by azeotropic dehydration in a hydrophobic organic solvent used in polymerization, or common forced draft furnace, dryer under reduced pressure, microwave dryer, high humidity drying using high temperature steam or the like may be employed after filtration of the hydrated gel-like polymer so that intended water content is attained. Preferably the method by azeotropic dehydration is used.Water content of the water-absorbing resin used in the present invention (defined by amount of water included in the water-absorbing resin/measured based on loss on drying at 180°C for 3 hours) is not particularly limited, however, it is selected so as to provide powder exhibiting fluidity even at room temperature in view of property of the resulting water-absorbing agent, and powder state with water content of more preferably 0.2 to 30% by weight, further preferably 0.3 to 15% by weight, and particularly preferably 0.5 to 10% by weight.

(9) Washing with lower-boiling point organic solvent

When a water-absorbing resin is produced by reversed phase suspension polymerization, polymerization was conducted using an organic solvent such as n-hexane, n-heptane or cyclohexane, followed by surface crosslinking, drying and agglomeration as described above. Therefore, there exists a problem involving presence of a slight amount of the organic solvent in conventional methods for production and commercially available products. The particulate water-absorbing agent can be obtained by means of the second production method (i.e. washing with lower-boiling point organic solvent).

In order to reduce the amount of residual organic solvent, a method in which the hydrated gel-like polymer obtained as described above is washed with an organic solvent or an inorganic solvent having a lower-boiling point may be used. This lower-boiling point organic solvent has a boiling point of, for example, preferably not lower than 0°C and not higher than 70°C, and more preferably not lower than 30°C and not higher than 50°C. Among them, acetone, dimethyl ether, methylene chloride and the like are preferred. Specifically, after filtrating the hydrated gel-like polymer, it may be washed with the lower-boiling point organic solvent described above, and dried by the hot air drying or the like. This washing may be carried out after the drying treatment. Alternatively, without or in combination with washing in this manner, the organic solvent may be removed in surface crosslinking treatment by heating at high temperature described later(the first production method:heat treatment) . Using amount of the solvent for washing may be usually 0.5 to 100 parts by weight, and preferably 1 to 10 parts by weight ,more preferably 2 to 5 parts by weight based on 1 part by weight the water-absorbing resin. The temperature may be not lower than the room temperature and not higher than the boilng point.

(10) Classification and particle size control, and absorption capacity control

A water-absorbing resin (cross-linked polymer) of the present invention is adjusted preferably to have specific particle size as powder to obtain the water-absorbing agent of the present invention. When the particle size is out of range described below, performance as a diaper can be hardly achieved. Particle size may be adjusted, when crosslinked polymer is produced by reversed phase suspension polymerization, by dispersion polymerization in particulate state and distribution drying, however, it may be adjusted to have a specific particle size by agglomerating. Also, it may be adjusted to have a specific particle size by pulverizing and classification after drying.

Particle diameter of the water-absorbing resin (cross-linked polymer) particle of the present invention is usually controlled to being not smaller than 180 $\mu$m and not larger than 420$\mu$m as mass median particle size (D50), with the lower limit being preferably 200 $\mu$m, more preferably 225$\mu$m, and particularly preferably 250 $\mu$m, while the upper limit being preferably 400 $\mu$m, more preferably 380 $\mu$m, and particularly preferably 350 $\mu$m; and ratio of particles with diameter of smaller than 150 $\mu$m and/or particles with diameter of larger than 850 $\mu$m is controlled to be 0 to 5% by weight, preferably 0 to 2% by weight, and more preferably 0 to 1% by weight in order to obtain the water-absorbing agent of the present invention.

Bulk density (specified by JIS K-3362(1998)) of the water-absorbing resin of the present invention is adjusted to be in the range of preferably 0.40 to 0.90 g/ml, and more preferably 0.50 to 0.80 g/ml to obtain the water-absorbing agent of the present invention. Ratio of particles with diameter of 150 to 600 $\mu$m is preferably 90 to 100% by weight, more preferably 95 to 100% by weight, and further more preferably 98 to 100% by weight in whole particles. Logarithmic standard deviation ($\sigma\zeta$) of particle size distribution is controlled to be preferably 0.20 to 0.50, more preferably 0.20 to 0.45, and particularly preferably 0.20 to 0.40. When the particle size is out of range, the ability of watertight is not achieved upon practical applications in absorbing articles such as a diaper.

Although the water-absorbing resin obtained in the present invention as described above is adjusted to fall within the aforementioned particle size, centrifuge retention capacity in a physiological saline solution before the surface crosslinking is preferably controlled to fall within the range of not lower than 32 g/g, with the lower limit being more preferably 35 g/g, more preferably 40 g/g, and particularly preferably 45 g/g, while the upper limit being more preferably 70 g/g, more preferably 65 g/g, and particularly preferably 60 g/g. When centrifuge retention capacity is out of range, improving of the property is not achieved after surface cross linking. Consequently, a diaper with superior property may not be obtained. Control of the absorption capacity may be perfected by controlling the polymerization conditions such as the internal crosslinking agent, and drying conditions.

(11) Surface crosslinking treatment (may be merely referred to as surface crosslinking)

A water-absorbing resin used in the water-absorbing agent of the present invention is obtained by the crosslinking polymerization and drying (or partial drying), and pulverization and classification as needed, followed by further subjecting the surface to a crosslinking (secondary crosslinking) treatment.

Although there exist a variety of crosslinking agents which may be used for subjecting the surface to crosslinking, in view of the property, a polyhydric alcohol compound, an epoxy compound, a polyvalent amine compound or a condensate thereof with a haloepoxy compound, an oxazoline compound, a mono-, di-, or polyoxazolidinone compound, a polyvalent metal salt, an alkylene carbonate compound or the like has been generally used. The surface crosslinking agents which may be used in the present invention are exemplified specifically, in US6228930, US6071976, US6254990 and the like. Examples thereof include e.g., polyhydric alcohol compounds such as mono-, di- or polyethyleneglycol, propyleneglycol, 1,3-propanediol, dipropyleneglycol, 2,3,4-trimethyl-1,3-pentanediol, glycerin, 2-butene-1,4-diol, 1,4-butanediol, 1,3-butanediol, 1,5-pentanediol, 1,6-hexanediol, 1,2-cyclohexanedimethanol and the like; epoxy compounds such as mono-, di- or polyethyleneglycol diglycidyl ether, glycidol and the like; oxazolidinone compounds such as 2-oxazolidinone; alkylene carbonate compounds such as ethylene carbonate, and the like, but not particularly limited thereto. To maximize effects of the present invention, it is preferable to essentially use a polyhydric alcohol among these surface crosslinking agents. A polyhydric alcohol having carbon atoms of 2 to 10, and preferably carbon atoms of 3 to 8 may be used.

Using amount of the surface crosslinking agent is generally 0.001 to 10 parts by weight, and preferably 0.01 to 5 parts by weight based on 100 parts by weight of the water-absorbing resin. In the present invention, it is preferred that water as a main component, particularly water alone is used as a solvent in the surface crosslinking. Amount of water used as the solvent may fall within the range of 0.5 to 20 parts by weight, and preferably 0.5 to 10 parts by weight based on 100 parts by weight of the water-absorbing resin. Although an organic solvent can be used in combination, residual solvent must be noted. Using amount of the solvent may be not higher than 0.5 part by weight, and preferably not higher than 0.1 part by weight, more preferably not higher than 0 part by weight based on 1 part by weight the water.

In the present invention, among a variety of mixing methods, preferable method is premixing of a surface crosslinking agent with water as needed, followed by spraying or drop-wise addition of the aqueous solution to the water-absorbing resin, and the spraying method is more preferable. Average particle diameter of the droplet to be sprayed is preferably 1 to 300 $\mu$m, and more preferably 2 to 200 $\mu$m.

The water-absorbing resin after mixing with the surface crosslinking agent is subjected to a heat treatment. Conditions for carrying out the heat treatment may be: temperature of the water-absorbing resin or heat medium temperature being usually not lower than 60°C and not higher than 280°C, with the lower limit being preferably 100°C, and more preferably 150°C, and the upper limit being preferably 250°C, and more preferably 240°C; and heating time being preferably 1 minute or longer and 2 hours or less,more preferably 10 minutes or longer and 1 hour or less, further preferably 15 minutes or longer and 45 minutes or less.

In order to obtain the water-absorbing agent of the present invention, suitably, a large amount of dispersion solvent is not used as in conventional reversed phase, but a heat treatment at high temperature without substantially using a dispersion solvent is preferred upon the surface crosslinking treatment according to the present invention. In other words, because water-absorbing resins according to conventional reversed phase polymerization were obtained by dispersion in an organic solvent followed by polymerization and surface crosslinking, it was found that dispersion solvent was easy to remain.

According to the present invention, a water-absorbing agent (first water-absorbing agent) in which a volatile organic material is not substantially present is obtained through heating the water-absorbing resin at high temperature, not lower than 150°C and not higher than 250°C, without using a dispersion solvent for the water-absorbing resin or an organic solvent for the surface crosslinking agent. Moreover, content of the acrylic acid dimer in the raw material is extremely slight as described above, and this dimer is decomposed into monomers during heating. Therefore, a water-absorbing agent (second water-absorbing agent) enabling suppression of increase in generation of the residual monomer through the following heating can be obtained. Heating at high temperature according to the present invention is preferred because it leads to removal of volatile organic compounds, and reduction of the amount of residual monomer which may be increased due to heat.

(12) Agglomeration

In order to obtain the water-absorbing agent of the present invention, agglomeration is carried out during or after polymerization. Agglomeration results in production of a water-absorbing agent which is further superior in water absorption speed and particle size, and is suited for practical applications e.g., as a diaper. Owing to the agglomeration, a water-absorbing agent that is more suited for practical applications in a diaper can be obtained.

The agglomeration method by reversed phase suspension as described above can be employed, as one alternative, in the present invention. However, because a problem of the odor due to the residual dispersion solvent was found in agglomeration by reversed phase suspension, an agglomeration method in which any dispersion solvent is not used in the present invention. In other words, an agglomeration method which has not been conventionally employed so often for reversed phase suspension polymerization, more specifically, a method in which water-absorbing resin particles dried and filtrated following polymerization are agglomerated using water is suitably employed in the present invention.

Accordingly, to obtain a particulate water-absorbing agent of the present invention, alternatively, the agglomeration step comprises heating with keeping the water content of not lower than 1% by weight and not higher than 10% by weight; and crush as needed, after subjecting the water-absorbing resin to the surface crosslinking treatment. Thus, the water-absorbing agent can be adjusted to have specific particle size as powder.

Water to be added to the water-absorbing resin may contain other additives such as chelating agents described later,components made from plants, antimicrobials, aqueous polymers, inorganic salts,etc. Content of them falls within the range of 0.001 to 50% by weight as a concentration in an aqueous solution.

In order to obtain the particulate water-absorbing agent of the present invention, particle diameter is controlled to fall within narrow range of not smaller than 200 $\mu$m and not larger than 400 $\mu$m, with the lower limit being preferably 225 $\mu$m, and more preferably 250 $\mu$m, while the upper limit being preferably 380 $\mu$m, and more preferably 350 $\mu$m, as mass median particle size (D50) of the particulate water-absorbing agent. In addition, ratio of particles with diameter of lower than 150 $\mu$m (defined by sieve classification) is controlled to be not lower than 0 % by weight and not higher than 5% by weight, with the upper limit being preferably 2% by weight, and more preferably 1% by weight.

Mass median particle size of the particulate water-absorbing agent obtained in agglomeration step of the present invention is controlled to be larger by preferably 5 to 30%, more preferably 7 to 25%, and still more preferably 9 to 20% than mass median particle size of the water-absorbing resin (cross-linked polymer) particles of the present invention.

In the particulate water-absorbing agent of the present invention, agglomerated particles account for 10% by weight or more and 100% by weight or less, with the lower limit being preferably 30% by weight, and more preferably 50% by weight in entire water-absorbing agent.

Additionally, in order to obtain the particulate water-absorbing agent of the present invention, its bulk density (specified by JIS K-3362 (1998)) is adjusted to be in the range of preferably 0.40 to 0.90 g/ml, and more preferably 0.50 to 0.80 g/ml. Further, the particulate water-absorbing agent of the present invention is produced to have ratio of particles with diameter of 150 to 600$\mu$m is preferably 90 to 100% by weight, more preferably 95 to 100% by weight, and further more preferably 98 to 100% by weight in whole particles. Logarithmic standard deviation ($\sigma\zeta$) of particle size distribution is controlled to be preferably 0.20 to 0.50, more preferably 0.20 to 0.45, and particularly preferably 0.20 to 0.40.

In the present invention, agglomerating is preferably performed by a method for spraying or drop-wise addition of water or an aqueous solution dissolved with other additives components to the water-absorbing resin, and a spraying method is more preferable. Droplet size to be sprayed is, as volume average particle diameter, preferably in the range of 0.1 to 300 $\mu$m, and more preferably in the range of 0.1 to 200 $\mu$m.

As agglomerating equipment used in agglomeration, those having strong mixing force are preferable. Suitable examples of the agglomerating equipment include e.g., a cylinder type mixer, a double wall conical mixer, a high speed agitation type mixer, a v-shaped mixer, a ribbon type mixer, a screw type mixer, a double-arm kneader, a crashing type kneader, a rotation type mixer, an air-flow type mixer, a turbulizer, a batch type Lödige mixer, a continuous type Lödige mixer and the like.

For promoting the agglomeration and reducing the amount of residual organic materials, the water-absorbing resin after mixing with water or the aqueous solution is subjected to a heat treatment. Heat treatment is performed, in view of agglomerating ratio or agglomerating strength, while maintaining water content (specified by weight loss in drying at 180°C for 3 hours) to be 1 to 10% by weight, more preferably 2 to 8% by weight and further preferably 2.5 to 6% by weight. Heating medium such as hot air can be used in heating, and heating temperature (temperature of heating medium or temperature of material) is preferably in the range of 40 to 120°C, more preferably in the range of 50 to 100°C. Heating time is preferably in the range of 1 minute to 2 hours. A suitable combination example of heating temperature and heating time is 60°C for 0.1 to 1.5 hour, and 100°C for 0.1 to 1 hour. Heating and the addition of water may be carried out by the same equipment or by separate equipment. Also, heating may be carried out with stirring or while standing still (without stirring), as long as temperature or water content can be controlled, but hardening (to give loose block shape) by heating while standing still (without stirring) is preferable. Preferably, in heating, curing may be performed by heating the water-absorbing resin added with water after heaping up of approximately 1 to 100 cm, more preferably 5 to 80 cm, and particularly preferably 10 to 70 cm. A hardened water-absorbing resin is then pulverized, and preferably classified to obtain intended agglomerate of the present invention.

In the agglomeration according to the present invention, because curing by heating is carried out after providing water alone or water as a main component (60 to 100%) for use as a binder, an absorbing core can be obtained which is not only safe but excellent because the agglomerate can be appropriately destroyed in practical applications, and mass median particle size can be controlled to fall within the range according to the present invention, even though the size is reduced by impact.

(13) Addition of chelating agent

To the water-absorbing agent of the present invention, a chelating agent, in particular, a polyvalent carboxylic acid and salts thereof can be formulated.

The chelating agent which may be used in the water-absorbing agent of the present invention is preferably a chelating agent with high ion blocking ability or chelating ability for Fe or Cu, specifically, a chelating agent with stability constant for Fe ion of not lower than 10, preferably not lower than 20. The chelating agent is further preferably an aminopolyvalent carboxylic acid and salts thereof, and particularly preferably an aminocarboxylic acid with not less than 3 carboxyl groups, preferably not less than 4 carboxyl groups, more preferably not less than 5 carboxyl groups, while the upper limit being polymer, usually not more than 100 carboxyl groups, preferably not more than 20 carboxyl groups, and salts thereof.

Specific examples of these polyvalent carboxylic acids include diethylenetriamine pentaacetic acid, triethylenetetramine hexaacetic acid, cyclohexane-1,2-diamine tetraacetic acid, N-hydroxyethyl ethylenediamine triacetic acid, ethyleneglycol diethylether diamine tetraacetic acid, ethylenediamine tetra propionic acetic acid, N-alkyl-N'-carboxymethyl aspartic acid,
N-alkenyl-N'-carboxymethyl aspartic acid and alkaline metal salts thereof; alkaline earth metal salts ammonium salts or amine salts thereof. Among these, diethylenetriamine pentaacetic acid, triethylenetetramine hexaacetic acid, N-hydroxyethyl ethylenediamine triacetic acid and salts thereof are most preferable.

Using amount of the chelating agent, in particular, the amino polyvalent carboxylic acid is as small as generally 0.00001 to 10 parts by weight based on 100 parts by weight of the water-absorbing resin, a main component, preferably 0.0001 to 1 part by weight, and more preferably 0.01 to 0.5 part by weight. The using amount over 10 parts by weight is not only uneconomical due to failure to achieve enough effect relative to using amount but also poses a problem of reducing absorption capacity and the like. On the other hand, the using amount less than 0.00001 part by weight does not provide sufficient addition effect.

(14) Other additives

In the present invention, the following (A) components made from plants, (B) polyvalent metal salts of organic acids, (C) inorganic fine particles (including (D) composite hydrated oxides) may be also added as minor components in addition to the chelating agent, by which various functions can be imparted to the water-absorbing resin of the present invention. Although using amount of these additives (A) to (D) and (E) may vary depending on the objectives and function to be imparted, exemplary amount of adding one of them is in the range of 0 to 10 parts by weight, preferably 0.001 to 5 parts by weight, and more preferably 0.002 to 3 parts by weight based on 100 parts by weight of the water-absorbing resin. In general, the amount less than 0.001 part by weight does not provide sufficient effect or additional function, while the amount over 10 parts by weight may not achieve effect to meet with the added amount, or may deteriorate absorbing performance.

(A) Components made from plants

A water-absorbing agent of the present invention can be added with a component made from a plant in the amount described above to fulfill deodorization effect. The component made from a plant to be used in the present invention is preferably at least one compound selected from polyphenol, flavone, derivatives thereof and caffeine. It is further preferable that the component made from plant is at least one kind of compound selected from tannin, tannic acid, stachyurus praecox, gall or gallic acid.

Plants containing the component made from a plant to be used in the present invention include, for example, Theaceae plant such as camellia, Hikasaki plant and Sprague; Gramineae plant such as rice plant, sasa-bamboo, bamboo, corn, wheat and the like; and Rubiaceae plant such as coffee.

Form of the components made from a plant to be used in the present invention includes plant extract (essential oil), plant itself (plant-ground powder), plant residue or extract residue as by-products generated in production processes from plant processing industry or foods processing industry, however, not particularly limited thereto. The above-mentioned components made from a plant are exemplified in EP1352927,US2004-48955 and the like.

(B) A polyvalent metal salt

To a water-absorbing agent of the present invention may be added a polyvalent metal salt, in particular, a polyvalent metal salt of organic acids in the amount described above to improve powder fluidity, and to prevent blocking in moisture absorption.

A polyvalent metal salt of an organic acid used and a method for mixing the polyvalent metal salt are exemplified in WO2004-69936, and examples of the polyvalent metal salt of organic acids used in the present invention, having carbon atoms of not less than 7 in a molecule include metal salts other than alkali metal salts of fatty acid, petroleum acid or polymeric acid,.

Illustrative examples of the organic acid composing the polyvalent metal salt of an organic acid include long chain or branched fatty acids such as caproic acid, octylic acid, octynoic acid, decanoic acid, lauric acid, myristic acid, palmitic acid, oleic acid, stearic acid and the like; petroleum acids such as benzoic acid, myristicinic acid, naphthenic acid, naphthoic acid, naphthoxyacetic acid and the like; polymeric acids such as poly (meth) acrylic acid, polysulfonic acid, and the like. The organic acid preferably has a carboxyl group in a molecule. More preferably, the organic acid may be a fatty acid such as caproic acid, octylic acid, octynoic acid, decanoic acid, lauric acid, myristic acid, palmitic acid, oleic acid, stearic acid, tallow acid or hydrogenated fatty acid of castor oil or the like. Still more preferably the organic acid may be a fatty acid without an unsaturated bond in a molecule, for example, caproic acid, octylic acid, decanoic acid, lauric acid, myristic acid, palmitic acid or stearic acid. Most preferably, the organic acid may be a long chain type fatty acid having not less than 12 carbon atoms in a molecule, without an unsaturated bond in a molecule such as laulic acid, myristic acid, palmitic acid or stearic acid.

(C) An inorganic fine particle

To a water-absorbing agent of the present invention is added an inorganic fine particle, in particular, an inorganic fine particle of non-dissolving in water to prevent blocking in moisture absorption. Silicon dioxide and silica acid (silicate salt) with average particle diameter, measured by a coulter counter method, of 0.001 to 200 $\mu$m are used. The deodorization ability can be achieved by above-mentioned zeolite .Amass ratio of the $SiO_2$ and $Al_2O_3$ in zeolite is preferably 1:1~100, more preferably 1:2~10.

(D) A composite hydrated oxide

To a water-absorbing agent of the present invention may be added a composite hydrated oxide containing zinc and silicon or zinc and aluminum (for example, exemplified in Japanese Patent Application No. 2003-280373) to add superior blocking ratio after moisture absorption (powder fluidity after the water-absorbing resin or the water-absorbing agent absorbs moisture) and further superior deodorization performance.

(E) A reducing agent

In a water-absorbing agent of the present invention may be preferably used a reducing agent, still more an inorganic reducing agent, and particularly a reducing agent containing sulfur or containing oxygen to prevent odor or coloring. Examples of the reducing agent to be used include sulfurous acid (sulfite) or sulfurous acid (hydrogen sulfite) and the like exemplified in US4863989.

(F) Others

Other additives such as an antimicrobial, an aqueous polymer, a water insoluble polymer, water, an organic fine particle, and the like may be added arbitrarily, as long as the water-absorbing agent of the present invention can be particularly obtained.

(15) A particulate water-absorbing agent of the present invention

The particulate water-absorbing agent is obtained by means of the first production method (i.e. heat treatment) . By the new water-absorbing agent, the problems of the present invention are solved. With the conventional water-absorbing resin and absorbing articles (especially diapers), there was a problem in practical applications as a diaper (especially as a diaper with high water-absorbing resin concentration) that rewet amount in a diaper was large, though many parameters of the water-absorbing resin were controlled. Moreover, it was a problem in certain instances that deodorization effect and reduction of the amount of residual monomers were insufficient, consequently a diaper with superior property may not be obtained. To solve the above-mentionedproblems (i.e. to provide water-absorbing articles which were suited for practical applications) in the present invention, the present inventors gave attention to content of the volatile organic compounds and/or the amount of residual monomer after heating in a water-absorbing agent (water-absorbing resin), which have conventionally not been focused, as means for solving the problems. The present inventors have also found that a new water-absorbing agent solves the above-mentioned problems by controlling particle size, absorption capacity, moreover content of the volatile organic compounds and/or the amount of residual monomer after heating in the water-absorbing agent.

A water-absorbing agent of the present invention is controlled to have (b) mass median particle size (D50) of being not smaller than 200 $\mu$m and not larger than 400 $\mu$m, with the lower limit being preferably 225$\mu$m, and particularly preferably 250 $\mu$m, while the upper limit being preferably 380 $\mu$m, and particularly preferably 350 $\mu$m. In addition, (c) ratio of particles with diameter of smaller than 150 $\mu$m is controlled to be not lower than 0 % by weight and not higher than 5% by weight, with the upper limit being preferably 3% by weight, more preferably 2% by weight, and particularly preferably 1% by weight. Particle size adjustment is preferably carried out before surface crosslinking, however, it may be adjusted by pulverizing, classification and agglomerating after surface crosslinking to give specific particle size. When mass median particle size is especially less than 200 $\mu$m and ratio of particles with diameter of smaller than 150 $\mu$m is larger than 5% by weight, a problem in connection with handling characteristics, particularly dust may be raised. Also, when it is used in absorbing articles such as a diaper, leakage from the top sheet could possibly occur. When mass median particle size is especially more than 400 $\mu$m, excellent absorption speed can not be achieved, thus, high property can not be obtained upon practical applications in absorbing articles such as a diaper.

The (a) centrifuge retention capacity (CRC) of the present invention may be not smaller than 32 g/g, with the lower limit being more preferably 35 g/g, still more preferably 40 g/g, and particularly preferably 45 g/g, while the upper limit being more preferably 70 g/g, still more preferably 65 g/g, and particularly preferably 60 g/g. When the absorption capacity is smaller than 32 g/g, high property can not be obtained in a diaper application.

The (d) content of volatile organic compounds as atmospheric concentration measured by a gas detector tube of the present invention is 0 ppm to 100 ppm, with the upper limit being preferably 50 ppm, more preferably 20 ppm, and still more preferably 5 ppm. This content means a content of volatile organic compounds in sealed air (atmospheric concentration) as determined by a measurement method of content of volatile organic compounds described later in Example (8). When the content of volatile organic compounds is higher than 100 ppm, odor of the organic compounds is generated in a diaper application, and thus, not only sense of discomfort is caused, but deodorization performance is deteriorated. Accordingly, high property can not be obtained. The volatile organic compounds means an organic solvent having boiling point at ordinary pressure of not higher than 150°C, preferably boiling point of 65 to 145°C, more preferably 75 to 130°C, and particularly preferably 80 to 125°C. In general, it may be any organic solvent used in manufacture, and examples thereof include e.g., the dispersion solvents essentially used in reversed phase polymerization, organic solvents may be used in the surface crosslinking as needed, as well as acetic acid, propionic acid, acrylic acid dimer and the like which are impurities of the monomer. It was found that remaining of these substances in a slight amount account for odor of the water-absorbing agent or inhibition of deodorization effect. Thus, these problems were dissolved by the present invention.

According to the present invention, (e) content of the residual monomer after heating the water-absorbing agent at 180°C for additional 3 hours is not lower than 0 ppm and not higher than 500 ppm, with the upper limit being preferably 300 ppm, and more preferably 100 ppm. The content of the residual monomer after heating for 3 hours of being higher than 500 ppm is not preferred. Because decomposition and the like of the acrylic acid dimer may be caused during practical applications (especially during practical applications under high temperature), or with additional reforming or treatment, leading to generation and increase of residual monomer, and to generation of an odor derived from the residual monomer, even though initial content of residual monomer is low.

In conventional reversed phase suspension polymerization of water-absorbing agents, as described above, polymerization and surface crosslinking, drying and agglomeration have been carried out in lower-boiling point organic solvent (for example, cyclohexane having boiling point of 80.7°C). Thus, it was found that a slight amount of organic solvent remains according to conventional methods for production and in commercially available products and the like, and that the residual organic solvent causes the odor in practical applications as a diaper. According to the present invention, such problems can be resolved by not substantially using organic solvent in surface crosslinking and agglomeration after reversed phase polymerization, and by heating at high temperature (preferably not lower than 150°C and not higher than 250°C), while controlling minor components in the monomer, particularly in acrylic acid (acetic acid, propionic acid, acrylic acid dimer and the like).

(16) Other properties of a particulate water-absorbing agent of the present invention

(f) Particles with diameter of 150 to 600 μm; (g) Logarithmic standard deviation

Bulk density (specified by JIS K-3362(1998)) of a water-absorbing agent of the present invention is adjusted to be preferably in the range of 0.40 to 0.90 g/ml and more preferably 0.50 to 0.80 g/ml. Ratio of (f) particles with diameter between 150 and 600 μm is preferably 90 to 100% by weight based on total particles, more preferably 95 to 100% by weight, and further preferably 98 to 100% by weight. Logarithmic standard deviation ($\sigma\zeta$) of particle size distribution (g) is in the range of preferably 0.20 to 0.50, more preferably 0.20 to 0.45, and particularly preferably 0.20 to 0.40. When particles without this range are used in a diaper, superior property may not be obtained. Because the water-absorbing agent of the present invention has narrow particle size distribution, handling characteristics (in particular, feeding characteristics) are favorable, and superior water absorption speed is provided. Therefore, when it is used as an absorbing article, superior liquid diffusibility is also achieved.

(h) Absorbency against pressure

A water-absorbing agent of the present invention has absorbency against pressure at 1.9 kPa (under load) in a 0.90% by weight aqueous sodium chloride solution of preferably not lower than 20 g/g, more preferably not lower than 25 g/g, still more preferably not lower than 30 g/g, and particularly preferably not lower than 35 g/g. When the absorbency against pressure is lower than 20 g/g, effect of the present invention may not be exerted. The upper limit is not particularly limited, however, approximately 60 g/g will be enough as the case may be, in view of elevation of the cost due to difficulty in production.

(i) Blocking ratio after moisture absorption

A water-absorbing agent of the present invention is superior in powder handling characteristics due to having low blocking ratio after moisture absorption described later in Example. Blocking ratio after moisture absorption is preferably 0 to 30% by weight, more preferably 0 to 20% by weight, further preferably 0 to 10% by weight and particularly preferably 0 to 5% by weight. Blocking ratio after moisture absorption over 30% by weight provides a problem such as difficulty in production of a diaper and the like, for example, owing to poor powder fluidity. The blocking ratio after moisture absorption falling within the above range can be attained by using the additives.

(j) Increased extractables by deterioration

Increased extractables by deterioration of the present invention is usually 0 to 15% by weight, more preferably 0 to 10% by weight, and still more preferably 0 to 5% by weight. When increased extractables by deterioration is over 15% by weight, stability of the water-absorbing agent against urine becomes insufficient and enough absorption ability cannot be fulfilled in long period practical application of the absorbing core.

(k) Increased ratio of extractables by deterioration

Increased ratio of extractables by deterioration of the present invention is usually 1 to 4 times, preferably 1 to 2 times, more preferably 1 to 1.5 times, and particularly preferably 1 to 1.3 times. When increased ratio of extractables by deterioration is over 3 times, stability of the water-absorbing agent against urine becomes insufficient and enough absorption ability cannot be fulfilled in long period practical application of the absorbing core.

(l) Absorption speed with vortex method

Absorption speed of the water-absorbing agent of the present invention is shorter than 60 seconds, preferably 1 to 55 seconds and more preferably 2 to 50 seconds. A water-absorbing agent with absorption speed over 60 seconds may not fulfill sufficient absorption ability when it used in absorbing cores such as a diaper.

(17) Shape of the particulate water-absorbing agent of the present invention

In general, particle shape of the water-absorbing resin (particulate water-absorbing agent)of the present invention obtained by reversed phase suspension polymerization exhibits nearly spherical shape, aggregated shape thereof, or aggregated shape derived from nearly spherical shapes, depending on the polymerization mechanism. This particle shape can be readily distinguished from irregular shape (pulverized shape or crushed shape) of the water-absorbing resin obtained by general aqueous polymerization. Fig. 1 illustrates an electron micrograph showing a water-absorbing resin forming particles with a shape like bunch of grapes through aggregation of nearly spherical shapes which are primary particles. Others,particles with wrinkles, particles with a perfect spherical body and particles with a sausage shape are exemplified. Fig. 2 illustrates an electron micrograph showing a water-absorbing resin forming rock-shaped particles through aggregation and fusion of nearly spherical shapes which are primary particles. Fig. 3 illustrates an electron micrograph showing a water-absorbing resin forming rice grain-shaped particles through aggregation and fusion of nearly spherical shapes which are primary particles. The water-absorbing resin obtained by reversed phase suspension polymerization has high bulk density of 0.9 g/mL in comparison with water-absorbing resins of irregular shape owing to the particle shape thereof. Therefore, it is advantageous in regard to transportation of the water-absorbing resin and in reduction in thickness of the absorbing core. Moreover, because of large surface area of the aggregates of nearly spherical shapes or aggregates derived from nearly spherical shapes, comparatively high liquid absorption speed can be attained. Therefore, it is advantageous in prevention of liquid leakage and skin irritation when used in absorbing cores such as a diaper.

(18) An absorbing article

Applications of the particulate water-absorbing agent of the present invention are not especially limited, however, the water-absorbing agent is used preferably in an absorbing core and an absorbing article.
The absorbing core is obtained using the particulate water-absorbing agent. The absorbing core means an absorbing material formed using the particulate water-absorbing agent and hydrophilic fibers as main components. Content of the water-absorbing agent (core concentration) in the absorbing core, based on total weight of the water-absorbing agent and hydrophilic fibers is preferably not lower than 20 and not higher than 100% by weight, with the lower limit being more preferably 30% by weight, and particularly preferably 40% by weight.
Further, an absorbing article of the present invention is equipped with the absorbing core, a surface sheet with liquid permeability and a back sheet with liquid non-permeability.
Absorbing articles of the present invention, in particular, a disposable diaper for a child, a disposable diaper for an adult or a sanitary napkin can be fabricated by, for example, preparing the absorbing core (absorbing substrate) through blending or sandwiching fiber substrates and the water-absorbing agent, followed by sandwiching the absorbing core between the substrate with liquid permeability (the surface sheet) and the substrate with liquid non-permeability (the back sheet) and mounting of elastic parts, diffusion layers, pressure sensitive adhesive tapes, and the like, if necessary. Such absorbing core is subjected to compression molding to have density of 0.06 to 0.50 g/cc and basis weight in the range of 0.01 to 0.20g/cm$^2$. Illustrative examples of the fiber substrate to be used include hydrophilic fibers, such as e.g., ground wood pulp, or cotton linters, crosslinked cellulosic fibers, rayon fibers, cotton fibers, wool fibers, acetate fibers, vinylon fibers, and the like. Preferably they are used as airlied.

A water-absorbing agent of the present invention is one exhibiting superior absorption properties. Specific examples of such absorbing article include sanitary goods starting from a disposable diaper for an adult, whose growth is significant recently, a diaper for a child, a sanitary napkin, so to speak a pad for incontinence, and the like, but not limited thereto. Because a water-absorbing agent present in an absorbing article of the present invention provides less rewet amount and significant dry feeling, loads of person wearing such goods and nursing staffs can be significantly reduced.

Examples

The present invention will be elucidated specifically with the following Examples and Comparative Examples below, without being limited to the following Examples. Various performances of a water-absorbing resin, a particulate water-absorbing agent (hereinafter, referred to as water-absorbing agent) and an absorbing article were measured by the following methods. In the following description of the measurement method, explanation will be made for cases in which a property of a water-absorbing agent is measured. Electrical equipment was always used under conditions of 100 V and 60 Hz in Examples. In addition, the water-absorbing resin, the water-absorbing agent and the absorbing article were used under conditions of 25°C ± 2°C and 50% RH (relative humidity), unless particularly specified. An aqueous solution of 0.90% by weight of sodium chloride was used as a physiological saline solution.

When moisture is absorbed by the water-absorbing agent (water-absorbing resin), measurement is carried out after drying under reduced pressure (for example, for about 16 hrs at 60 to 80°C), as appropriate, to equilibrium moisture content(2 to 8% by weight, about 5% by weight) of the water-absorbing agent (water-absorbing resin). When a water-absorbing agent (water-absorbing resin), a diaper, or a water-absorbing agent(a water-absorbing resin) taken out of a diaper on the market which may absorb moisture on distribution is used in a comparison test, comparison may be made after drying under reduced pressure (for example, for about 16 hrs at 60 to 80°C) as appropriate to equilibrium moisture content (2 to 8% by weight, about 5% by weight) of the water-absorbing agent (water-absorbing resin).

(1) Centrifuge Retention Capacity (CRC) for a physiological saline solution (an aqueous solution of 0.90% by weight of sodium chloride)

A water-absorbing agent of 0.20 g was uniformly put in a bag (60 mm x 85 mm) made of unwoven fabric and immersed in a physiological saline solution controlled at 25 ± 2°C. The bag containing the water-absorbing agent was taken out of the saline solution after 30 minutes and subjected to dewatering for 3 minutes at 250 G (250 x 9.81 m/sec$^2$) using a centrifuge (Model H-122 small size centrifuge made by Kokusan Corporation) and then weighed to determine weight $W_2$ (g) . Weight $W_1$ (g) of the bag then was measured after similar operation without using any water-absorbing agent. Centrifuge Retention Capacity (g/g) was calculated from the weights $W_1$ and $W_2$ according to the following formula.

$$\text{Centrifuge Retention Capacity (g/g)} = \{(\text{weight } W_2 \text{ (g)} - \text{weight } W_1 \text{ (g)}\}/\text{weight of water-absorbing agent (g)} - 1$$

(2) Absorbency Against Pressure at 1.9 kPa (AAP 1.9 kPa) in a physiological saline solution

A water-absorbing agent of 0.900 g was uniformly scattered on a 400-Mesh wire mesh made of stainless steel (mesh size: 38 μm) welded to the bottom end face of a plastic support cylinder with inner diameter of 60 mm. A piston (cover plate), which has outer diameter a little smaller than 60 mm, forms no gap against the inner surface of the support cylinder and can move up and down smoothly, was mounted on the water-absorbing agent. Total weight $W_3$ (g) of the support cylinder, the water-absorbing agent and the piston was measured. A load adjusted weight to press the water-absorbing agent uniformly at 1.9 kPa including the weight of the piston was mounted on the piston, thereby completing a set of measuring apparatus. A glass filter (mesh size: 40~50μm) with, diameter of 90 mm and thickness of 5 mm was placed in a Petri dish with diameter of 150 mm and a physiological saline solution controlled at 25 ± 2°C was poured up to the same level as the upper surface of the glass filter. A sheet of filter paper with diameter of 9 cm (No.2 from Toyo Roshi Kaisha Ltd.) was placed on the surface of the glass filter to be entirely wetted, and then excess solution over the wetted filter paper was removed.

The set of the measuring apparatus was placed on the wetted filter paper and the liquid was absorbed with the water-absorbing agent under load. The liquid level was kept constant by adding the liquid when the liquid surface became lower than the upper surface of the glass filter. The set of the measuring apparatus was lifted up after an hour and weight

$W_4$ (g) (the total weight of the support cylinder, the swollen water-absorbing agent and the piston) excluding the load was measured again. The absorbency against pressure (g/g) was calculated from the weights $W_3$ and $W_4$ according to the following formula.

$$\text{Absorbency Against Pressure (g/g)} = \{\text{weight } W_4 \text{ (g)} - \text{weight } W_3 \text{ (g)}\}/\text{weight of water-absorbing agent (g)}$$

(3) Mass median particle size (D50), logarithmic standard deviation ($\sigma\zeta$) and percentage by weight of particles less than 150 $\mu$m in diameter

A water-absorbing agent was subjected to sieve classification using JIS standard sieves (JIS Z-8801-1 (2000)) having mesh opening size of 850 $\mu$m, 710 $\mu$m, 600 $\mu$m, 500 $\mu$m, 425$\mu$m, 300 $\mu$m, 212$\mu$m, 150 $\mu$m, 106 $\mu$m and 45 $\mu$m, and percentage by weight of particles less than 150 $\mu$m in diameter was measured, while oversize percentages R at each particle size were plotted on a logarithmic probability paper. Particle size corresponding to R = 50% by weight was thus determined as mass median particle size (D50). Logarithmic standard deviation ($\sigma\zeta$) is represented by the following formula, wherein smaller value of $\sigma\zeta$ means narrower particle-size distribution.

$$\sigma\zeta = 0.5 \times \ln (X_2 / X_1)$$

(wherein $X_1$ and $X_2$ are particle diameters for R = 84.1% by weight and R = 15.9% by weight, respectively)

For sieve classification, a water-absorbing agent of 10.00 g is charged into each of the JIS standard mesh sieves (The IIDA TESTING SIEVE: inner diameter of 80 mm) and sieved for classification for 5 minutes using a Ro-tap type sieve shaker (Model ES-65 sieve shaker from Iida Seisakusho Co., Ltd.).

Mass median particle size (D50) means particle diameter for a standard sieve, corresponding to 50% by weight based on the whole particles, when sieving is carried out by standard sieves with particular meshes as described in US5051259 and the like.

(4) Extractable polymer

First of all, preparation for measurement of extractable polymer will be described. A pH electrode was calibrated with buffer solutions of pH 4.0, pH 7.0 and pH 10.0. A physiological saline solution of 50 ml, prepared beforehand for a blank test, was poured into a 100 ml glass beaker and added in dropwise with a 0.1 mol/L aqueous solution of sodium hydroxide until pH 10, while stirring with a stirrer chip with length of 30 mm, to determine titration amount $V_{ab}$ (ml) of the aqueous solution of sodium hydroxide for a blank test. The saline solution was further added in dropwise with a 0.1 mol/L aqueous solution of hydrochloric acid until pH 2.7, while stirring, to determine titration amount $V_{bb}$ (ml) of hydrochloric acid for a blank test.

A physiological saline solution of 200 ml, prepared beforehand, was poured into a 250 ml polypropylene cup with a lid and added with 1.0g (= m(g)) of a water-absorbing agent obtained in Examples or Comparative Examples to be described later. The saline solution was then stirred with a stirrer with length of 30 mm and diameter of 8 mm at 500 $\pm$ 50 rpm for one hour or 16 hours to extract extractable polymer. After stirring for one hour or 16 hours, the saline solution was filtrated with a filter paper (No.2, made by Toyo Roshi Kaisha Ltd., retaining particle size of 5 $\mu$m according to JIS P 3801 (1995)) to obtain a filtrate.

Thus obtained filtrate of 20 ml (recorded as F (ml)) was poured into a 100 ml glass beaker and added with a physiological saline solution to obtain 50 ml of a filtrate for titration. When thus obtained filtrate is less than 20 ml, the total amount of thus obtained filtrate was recorded as F (ml) and added with a 0.90% by weight aqueous solution of sodium chloride to obtain 50 ml of a filtrate for titration.

Subsequently, the filtrate for titration was added in dropwise with a 0.1 mol/L aqueous solution of sodium hydroxide, while stirring with a cylinder-type stirrer with length of 30 mm and diameter of 8 mm until pH 10, to determine titration amount $V_a$ (ml) of the aqueous solution of sodium hydroxide. The filtrate was further added in dropwise with a 0.1 mol/L aqueous solution of hydrochloric acid, while stirring, until pH 2.7, to determine titration amount $V_b$ (ml) of the aqueous solution of hydrochloric acid. Extractable polymer content (%) is calculated by the following formula.

$$\text{Extractable polymer content (\%)} = \{(W_a + W_b)/m\} \times 100$$

Wherein $W_a$ (g) is relative weight of units having an acid group in extractable polymer included in a water-absorbing agent, and $W_b$ (g) is relative weight of units having a carboxylate group neutralized by an alkali metal in extractable polymer included in the water-absorbing agent. Each of them is calculated by the following formulae.

$$W_a \ (g) \ = \ N_a \ x \ 72 \ x \ 200/F$$

$$W_b \ (g) \ = \ N_b \ x \ 94 \ x \ 200/F$$

Wherein the value 72 is weight per 1 mole of a repetition unit in polyacrylic acid, and when a monomer with an acid group other than acrylic acid is copolymerized, the value is altered to average weight of a repetition unit including such a monomer. The value 94 is weight per 1 mole of a repetition unit in polysodium acrylate and it is altered, as appropriate, when a monomer having an acid group other than acrylic acid is copolymerized, or when potassium, lithium or the like is used instead of sodium as an alkali metal salt.

$N_a$ (mol) is number of moles of an acid group of extractable polymer in a filtrate and $N_b$ (mol) is number of moles of a carboxylate group, neutralized by an alkali metal, of extractable polymer in the filtrate. Each of them is calculated by the following formulae.

$$N_a \ (mol) \ = \ \{ (V_a \ - \ V_{ab})/1000 \} \ x \ 0.1$$

$$N_b \ (mol) \ = \ N_1 \ - \ N_a$$

Wherein $N_1$ (mol) is number of total moles of extractable polymer in a filtrate to be measured and is calculated by the following formula.

$$N_1 \ (mol) \ = \ \{ (V_b \ - \ V_{bb})/1000 \} \ x \ 0.1$$

The contents of extractable polymer determined by the above formulae were differentiated as extractable polymer content for one hour (%) on a filtrate obtained by stirring a physiological saline solution for one hour, and extractable polymer content for 16 hours (%) on a filtrate obtained by stirring a physiological saline solution for 16 hours.

(5) Evaluation of resistance to urine

A physiological saline solution prepared beforehand was added with L-ascorbic acid to give 0.05% by weight so as to obtain mimicked artificial urine for deterioration test. Specifically, 0.50 g of L-ascorbic acid was dissolved in 999.5 g of a physiological saline solution to prepare mimicked artificial urine for deterioration test.

Mimicked artificial urine for deterioration test of 25 ml was poured into a 250 ml polypropylene cup with a lid and added with 1.0 g of a water-absorbing agent obtained in Examples or Comparative Examples to be described later to form swollen gel. After the cup was sealed with the lid, the swollen gel was left for standing in atmosphere of 37°C for 16 hours.

After 16 hours, 175 ml of the physiological saline solution was added and then extractable polymer after deterioration was extracted from the hydrated gel by stirring for one hour similarly as in the previous item (4) with a cylinder-type stirrer with length of 30 mm and diameter of 8 mm.

After extraction by stirring for one hour, the saline solution was filtrated and pH-titrated by the same method as in the previous item (4) to determine extractable polymer content after deterioration $Ex_1$ (% by weight) using the same formulae as in the previous item (4). To compare absolute amount of increased extractable polymer content by deterioration, for evaluation of resistance to urine, increased extractables by deterioration (% by weight) was calculated by the following formula.

$$\text{Increased extractables by deterioration (\% by weight)}$$
$$= \ Ex_1 \ - \ Ex_2$$

wherein $Ex_2$ is extractable polymer content for one hour (% by weight).

To compare extractable polymer content formed by deterioration with extractable polymer content before deterioration, for evaluating resistance to urine, increased ratio of extractables by deterioration (quotient) was calculated by the following formula.

$$\text{Increased ratio of extractables by deterioration} = Ex_1 / Ex_2$$

(6) Evaluation of absorption speed (Vortex method)

To a 0.90% by weight aqueous solution of sodium chloride (physiological saline solution) prepared previously of 1000 parts by weight was added 0.02 part by weight of a brilliant blue-FCF, a food additive, and kept at 30°C. The physiological saline solution of 50 ml was poured into a 100 ml beaker and added with 2.0 g of a water-absorbing agent obtained in Examples or Comparative Examples to be described later, while stirring at 600 rpm with a cylinder-type stirrer with length of 40 mm and diameter of 8 mm, to measure absorption speed (second). Absorption speed (second) was measured in terms of time required for the test liquid to completely cover the stirrer chip as the water-absorbing agent absorbs the physiological saline solution, which was measured with end point determined according to the standard described in JIS K 7224 (1996) "Testing method for water absorption speed of super absorbent resins - Description".

(7) Blocking ratio (Fluidity) after moisture absorption (% by weight)

A particulate water-absorbing agent of 2 g described later was uniformly scattered on the bottom of an aluminum cup with diameter of 52 mm and height of 22 mm and quickly put in a humidity-controllable incubator (PLATIOOUS LUCIFER PL-2G from ESPEC Corp.) controlled beforehand at 25°C and 90% relative humidity, and left for standing for 60 minutes. The moisture-absorbed water-absorbing agent was then transferred to a JIS standard sieve with diameter of 7.5 cm and 2000 $\mu$m of mesh open. When the moisture-absorbed water-absorbing agent was adhered to aluminum cup too rigidly to do so, the moisture-absorbed water-absorbing agent accompanied by blocking should be detached from the cup and transferred to the sieve very carefully not to destroy the block. The transferred water-absorbing agent to the sieve was immediately sieved for 8 seconds using a sieve shaker (IIDA SIEVE SHAKER, TYPE: ES-65, SER. No. 0501). Weight $W_5$ (g) of the oversize water-absorbing agent left on the sieve and weight $W_6$ (g) of the undersize water-absorbing agent passed through the sieve were measured. Blocking ratio after moisture absorption (% by weight) was calculated by the following formula. The lower blocking ratio after moisture absorption means the water-absorbing agent that is more superior in fluidity after moisture absorption, with improved powder handling nature.

$$\text{Blocking ratio after moisture absorption (\% by weight)} = \{\text{weight } W_5 \text{ (g)}/(\text{weight } W_5 \text{ (g)} + \text{weight } W_6 \text{ (g)})\} \times 100$$

(8) Content of volatile organic compounds(ppm)

A water-absorbing resin of 6.00g was uniformly spread in a glass Petri dish (Code: 305-07, external diameter x height = 120 mm x 25 mm, described in GENERAL CATALOGUE A-1000 (published in 2003) published by SOGO LABORATORY GLASS WORKS CO., LTD). Then, the water-absorbing agent was covered by one piece of breathable and liquid permeable heatlon paper (Nangoku Pulp Technical Corporation, type: GSP-22) cut into circular (diameter: 116 mm). When heatlon paper can not be used, nonwoven fabric may be substituted. Circumference of the heatlon paper (or nonwoven fabric) was fixed on inner wall of the glass Petri dish with a tape (10 mm x 10 mm) at three sites. One edge of a 3 L sniffing bag (manufactured by OMI ODORAIR SERVICE Corporation) was opened, and after placing the glass Petri dish including the spread water-absorbing resin therein, opened part of the sniffing bag was closed with an adhesive tape not to leave any space. After depressurizing inside of the sniffing bag once from a glass tube attached to the sniffing bag, odorless air of 1.2 L was fed therein, and subsequently, 30 ml of a 0.90% by weight aqueous solution of sodium chloride (physiological saline solution) adjusted to a temperature of $25 \pm 2$°C was poured at once to the dish in the sniffing bag, while preventing contamination of the ambient air, using a glass funnel connected to a Teflon (registered trade name) tube. Then, the water-absorbing agent was uniformly swollen, and the bag was sealed hermetically with a silicon rubber stopper. The bag was left to stand in an incubator at 37°C to permit swelling. After leaving to stand in an incubator at 37°C for 60 minutes, the bag was taken out, and subsequently was left to stand at room temperature (20 to 30°C). After leaving to stand at room temperature for 10 minutes, the silicon rubber stopper was removed, and atmospheric concentration was measured using a gas sampler (manufactured by Gastec Corporation, GV-100S) and a gas detector tube (manufactured by Gastec Corporation, No. 102L) while preventing contamination of the ambient air. Distance between tip of the gas detector tube and the swollen water-absorbing agent was kept to be 5 to 10 cm during the time period from initiation to termination of the measurement. Measurement using the gas sampler and gas detector tube was carried out according to the method specified for the gas detector tube. Thus determined atmospheric concentration was defined as volatile organic compounds content (ppm). In this measurement method, as the case may be, the gas detector tube may execute detection to result in change of color even though similar operation is conducted using a physiological saline solution alone without using a water-absorbing agent. In such a case, correction was performed by subtracting a blank value detected in the indicator range yielded when a physiological saline solution alone was used (detection limit: 10 ppm).

(9) Evaluation of an absorbing core performance (Rewet amounts after 10 minutes)

An absorbing core was prepared using a water-absorbing agent obtained in Examples or Comparative Examples described later and subjected to a rewet test in order to evaluate performance as an absorbing core.

To begin with, a method for preparation of an absorbing core for evaluation is shown below.

A water-absorbing agent to be described later of 1 part by weight and crushed wood pulp of 2 parts by weight were subjected to dry mixing using a mixer. Thus obtained mixture was spread on a wire screen of 400-Mesh (mesh size: 38 $\mu$m) to form a web with diameter of 90 mm. The web was pressed under pressure of 196.14 kPa (2 kgf/cm$^2$) for 1 minute to obtain an absorbing core for evaluation with basis weight of 0.05 g/cm$^2$.

Subsequently, a method for evaluation of rewet amount after 10 minutes is shown below. The absorbing core for evaluation was placed on the bottom of a Petri dish with inner diameter of 90 mm made of stainless steel (SUS), and nonwoven fabric with diameter of 90 mm was placed thereon. Then, 30 ml of the mimicked artificial urine for deterioration test which had been prepared previously, used in the above item (5); evaluation of resistance to urine, was then poured over the nonwoven fabric and subjected to absorption for 10 minutes under conditions of no load. Thereafter, 30 sheets of filter paper with diameter of 90 mm (No. 2 from Toyo Roshi Kaisha Ltd.), whose total weight ($W_7$ (g)) was measured-beforehand, were placed, and a piston and a load (total weight of the piston and load being 20 kg) with diameter of 90 mm were placed on the filter paper so as to press uniformly the absorbing core, the nonwoven and the filter paper. The filter papers were made to absorb rewet liquid, while pressing for 5 minutes. The 30 sheets of the filter paper were then weighed ($W_8$ (g)) to calculate rewet amount after 10 minutes.

$$\mathtt{Rewet~amount~after~10~minutes~(g)~=~W_8~(g)~-~W_7~(g)}$$

(10) Amount of residual monomer

A water-absorbing agent of 1.000 g was dispersed in 200 ml of a physiological saline solution, which was stirred with a magnetic stirrer (500 rpm) for 1 hour, and the resulting swollen gel was filtrated through a filter (0.45 $\mu$m). The amount of residual monomer was quantitatively determined by analyzing thus obtained filtrate on liquid chromatography (HPLC). Measurement was carried out by a specific measurement method according to Measurement method of the amount of residual monomer of superabsorbent materials (edana RESIDUAL MONOMERS 410.2-02) recommended by EUROPEAN DISPOSABLES AND NONWOVENS ASSOCIATION (EDANA) .

[Production Example of acrylic acid 1]

Commercially available acrylic acid (containing 2000 ppm acrylic acid dimer, 500 ppm acetic acid and 500 ppm propionic acid) was supplied into column the bottom of a high boiling point impurities-separating column having 50 dual-flow perforated plates,and then distilled in a reflux ratio of 1 to remove maleic acid and a dimmer of acrylic acid (acrylic acid dimer) and crystalized. Accordingly, acrylic acid (containing 20 ppm acrylic acid dimer, 50 ppm acetic acid and 50 ppm propionic acid) was obtained.

[Production Example of acrylic acid 2]

Commercially available acrylic acid (containing 2000 ppm acrylic acid dimer, 500 ppm acetic acid and 500 ppm propionic acid) was supplied into column the bottom of a high boiling point impurities-separating column having 20 dual-flow perforated plates,and then distilled in a reflux ratio of 1 to remove maleic acid and a dimmer of acrylic acid (acrylic acid dimer) and crystalized. Accordingly, acrylic acid (containing 20 ppm acrylic acid dimmer, 500 ppm acetic acid and 500 ppm propionic acid) was obtained.

[Production of aqueous solution of sodium acrylate]

Acrylic acid in an amount of 1390 g was neutralized with 48% sodium hydroxide according to Example 9 in US5210298 at 20 to 40°C to obtain 100 % by mol neutralized sodium acrylate at the concentration of 37 % by weight.

[Example 1] - not encompassed by the claimed invention

Into a 2-L four-neck separable flask equipped with a stirrer, a reflux condenser, a thermometer, an inlet tube for nitrogen gas and a dropping funnel was charged 1.0 L of cyclohexane, and thereto was added 3.8 g of sucrose fatty acid ester (manufactured by DAI-ICHI KOGYO SEIYAKU CO.,LTD., DK-ester F-50, HLB = 6) as a dispersant which was dissolved therein. Dissolved oxygen was degassed by bubbling nitrogen gas. In another flask, 84.6 g of sodium acrylate that is a neutralized product of acrylic acid of Production Example 1, 21.6 g of acrylic acid of Production Example 1 and 0.016 g of N,N'-methylenebisacrylamide were dissolved in 197 g of ion exchanged water, and therein was dissolved 0.4 g of hydroxyethyl cellulose (manufactured by Daicel Chemical Industries, Ltd., HEC-Daicel EP-850) to prepare an aqueous solution of monomer having monomer concentration of 35% by weight. To this aqueous solution of monomer was added 0.15 g of potassium persulfate which was dissolved therein. Thereafter, dissolved oxygen was degassed by bubbling nitrogen gas. Then the aqueous solution of monomer in this flask was added to the separable flask to allow for dispersion by stirring. Thereafter, polymerization reaction was initiated by raising the bath temperature to 60°C, followed by keeping this temperature for 2 hours. After completing the polymerization, water included in the hydrated gel was removed by azeotropic dehydration with cyclohexane, followed by filtration, drying under reduced pressure at 80°C to obtain spherical polymer powder. Thus resulting polymer powder had water content of 5.6%.

With 100 parts by weight of the polymer was mixed 3.53 parts by weight of a surface crosslinking agent consisting of 0.5 part by weight of propyleneglycol, 0.03 part by weight of ethyleneglycol diglycidyl ether, 0.3 part by weight of 1,4-butanediol and 2.7 parts by weight of water. The mixture was subjected to a heat treatment at 210°C for 45 min. After surface crosslinking, 3 parts by weight of water was further added to the mixture, which was heated hermetically at 60°C for 30 minutes. Agglomerated particulate water-absorbing agent (1) was obtained by classification for 850 μm. Centrifuge retention capacity, absorbency against pressure at 1.9 kPa, particle size distribution, mass median particle size (D50), logarithmic standard deviation (σζ) and percentage by weight of particles having diameter of smaller than 150 μm, extractable polymer, evaluation of resistance to urine, absorption speed, blocking ratio after moisture absorption, content of volatile organic compound, and the amount of residual monomer after heating at 180°C for 3 hours of thus obtained particulate water-absorbing agent (1) are shown in Table 1 and Table 2.

[Comparative Example 1]

Into a 2-L four-neck separable flask equipped with a stirrer, a reflux condenser, a thermometer, an inlet tube nitrogen gas and a dropping funnel was charged 1.0 L of cyclohexane, and thereto was added 3.8 g of sucrose fatty acid ester (manufactured by DAI-ICHI KOGYO SEIYAKU CO.,LTD., DK-ester F-50, HLB = 6) as a dispersant which was dissolved therein. Dissolved oxygen was degassed by bubbling nitrogen gas. Separately in another flask, 84.6 g of sodium acrylate that is a neutralized product of commercially available acrylic acid (containing 2000 ppm acrylic acid dimer, 500 ppm acetic acid and 500 ppm propionic acid), 21.6 g of commercially available acrylic acid and 0.016 g of N,N'-methylenebisacrylamide were dissolved in 197 g of ion exchangedwater, and therein was dissolved 0.4 g of hydroxyethyl cellulose (manufactured by Daicel Chemical Industries, Ltd., HEC-Daicel EP-850) to prepare an aqueous solution of monomer having monomer concentration of 35% by weight. To this aqueous solution of monomer was added 0.15 g of potassium persulfate which was dissolved therein. Thereafter, dissolved oxygen was degassed by bubbling nitrogen gas. Then the aqueous solution of monomer in this flask was added to the saparable flask to allow for dispersion by stirring. Thereafter, polymerization reaction was initiated by raising the bath temperature to 60°C, followed by keeping this temperature for 2 hours. After completing the polymerization, water was removed from the hydrated gel-like polymer by azeotropic dehydration.

After adding 85.0 mg of ethyleneglycol diglycidyl ether as a surface crosslinking agent to dehydrated hydrated gel-like polymer, surface crosslinking treatment was carried out by keeping bath at 80°C for 2 hours. After agglomeration by reversed phase suspension (organic solvent: cyclohexane), the mixture was filtrated, followed by drying under reduced pressure at 80°C to obtain a comparative particulate water-absorbing agent (1). Results of evaluation of thus obtained comparative particulate water-absorbing agent (1) in a similar manner to Example 1 are shown in Table 1 and Table 2. The amount of residual monomer before heating in Comparative Example 1 was 50 ppm.

[Example 2]

To 100 parts by weight of the particulate water-absorbing agent (1) obtained in Example 1 was added and mixed (dry blended) 0.3 part by weight of fine particulate silicon dioxide (manufactured by NIPPON AEROSIL CO., LTD., AEROSIL ® 200 (mean particle size of primary particles: 12 nm)) to obtain a particulate water-absorbing agent (2). Results of evaluation of thus obtained particulate water-absorbing agent (2) in a similar manner to Example 1 are shown in Table 1 and Table 2.

[Example 3] - not encompassed by the claimed invention

With 100 parts by weight of the particulate water-absorbing agent (1) obtained in Example 1 was mixed by spraying 2 parts by weight of an aqueous solution comprising diethylenetriamine pentaacetatic acid aqueous solution and a 15% by weight aqueous solution of extract from plant leaves of Theaceae plant (Trade Name: FS-80MO, Supplier: Shiraimatu Shinyaku Co., Ltd., Address: 37-1 Ugawa, Minakuchi-Cho, Kouka-Gun, Shiga-Ken, Japan) (amount of each component being adjusted so that diethylenetriamine pentaacetatic acid and the 15% by weight aqueous solution of extract from plant leaves of Theaceae plant became 50 ppm by weight and 0.1% by weight by weight relative to the water-absorbing agent (1), respectively) . The resultant mixture was cured at 60°C for 1 hour, and thereto was then added and mixed (dry blended) 0.3 part by weight of fine particulate calcium stearate (manufactured by Kanto Chemical Co., Inc.) to obtain a particulate water-absorbing agent (3). Results of evaluation of thus obtained particulate water-absorbing agent (3) in a similar manner to Example 1 are shown in Table 1 and Table 2.

[Example 4] - not encompassed by the claimed invention

To evaluate an absorbing core performance of the water-absorbing agent (1) obtained in Example 1, an absorbing core for evaluation (1) was prepared according to the method for evaluation of an absorbing core performance in the above item (9), to measure rewet amount after 10 min. Results are shown in Table 3.

[Examples 5 and 6] - Example 6 is not encompassed by the claimed invention

The same procedures as in Example 4 were repeated except for using the particulate water-absorbing agents (2) and (3) obtained in Examples 2 and 3 instead of the particulate water-absorbing agent (1) used in Example 4, to obtain absorbing cores for evaluation (2) and (3), respectively. Rewet amounts of thus obtained absorbing cores for evaluation (2) and (3) were measured. The results are shown in Table 3.

[Comparative Example 2]

The same procedures as in Example 4 were repeated except for using the comparative particulate water-absorbing agent (1) obtained Comparative Example 1 instead of the particulate water-absorbing agent (1) used in Example 4, to obtain a comparative absorbing core for evaluation (1). Rewet amount of thus obtained comparative absorbing core for evaluation (1) was measured. The results are shown in Table 3.

[Comparative Example 3]

The same procedures as in Example 1 were repeated except for using the acrylic acid obtained in Production Example 2 instead of the acrylic acid obtained in Production Example 1, neutralizing the acrylic acid to obtain sodium acrylate, followed by polymerization similarly to Example 1. Results from thus obtained comparative particulate water-absorbing agent (3) are shown in Table 1 and Table 2.

[Comparative Example 4]

The same procedures as in Example 1 were repeated except for using the commercially available acrylic acid directly, which was neutralized to obtain sodium acrylate, followed by polymerization similarly to Example 1. Results from thus obtained comparative particulate water-absorbing agent (4) are shown in Table 1 and Table 2.

Table 1

Table 1

| | Particulate water absorbing agent | Centrifuge retention capacity (g/g) | Absorbency against pressure at 1.9 Kpa (g/g) | Extractable polymer content for 16 hours (%) | Increased extractables by deterioration (%) | Increased ratio of extractables by deterioration | Water absorption speed (sec) | Fluidity after moisture absorption (%) | Content of volatile orpnic material (ppm) | Amount of residual monomer after heating at 180°C for 3 hrs (ppm) |
|---|---|---|---|---|---|---|---|---|---|---|
| Example 1 | Particulate water absorbing agent(1) | 34 | 33 | 22 | 25 | 3.1 | 35 | 31 | 1 or less | 300 |
| Example 2 | Particulate water absorbing agent(2) | 34 | 32 | 22 | 25 | 3.1 | 35 | 0 | 1 or less | 300 |
| Example 3 | Particulate water absorbing agent (3) | 34 | 33 | 22 | 3 | 1.2 | 46 | 0 | 1 or less | 300 |
| Comparative Example 1 | Comparative particulate water absorbing agent (1) | 34 | 30 | 22 | 25 | 3.1 | 35 | 36 | 170 | 1500 |
| Comparative Example 3 | Comparative particulate water absorbing agent (3) | 34 | 33 | 22 | 25 | 3.1 | 35 | 31 | 120 | 300 |

(continued)

| | Particulate water absorbing agent | Centrifuge retention capacity (g/g) | Absorbency against pressure at 1.9 Kpa (g/g) | Extractable polymer content for 16 hours (%) | Increased extractables by deterioration (%) | Increased ratio of extractables by deterioration | Water absorption speed (sec) | Fluidity after moisture absorption (%) | Content ofvolatile orpnic material (ppm) | Amount of residual monomer after heating at 180°C for 3 hrs (ppm) |
|---|---|---|---|---|---|---|---|---|---|---|
| Comparative Example 4 | Comparative particulate water absorbing agent (4) | 34 | 33 | 22 | 25 | 3.1 | 35 | 31 | 120 | 600 |
| Note) Amount of residual monomer before heating in Comparative Example 1 is 50 ppm. | | | | | | | | | | |

EP 1 796 831 B1

Table 2

| | Water absorbing resin composition | Particle size distribution | | | | | | | | | | Mass median particle size D50 (μm) | Logarithmic standard deviation (σζ) |
| --- | --- | --- | --- | --- | --- | --- | --- | --- | --- | --- | --- | --- | --- |
| | | Not smaller than 850μm (% by weight) | Not smaller than 710 μm Smaller than 850 μm (% by weight) | Not smaller than 600 μm Smaller than 710 μm (% by weight) | Not smaller than 500 μm Smaller than 600 μm (% by weight | Not smaller than 425 μm Smaller than 500 μm (% by weight) | Not smaller than 300 μm Smaller than 425 μm (% by weight) | Not smaler than 212 μm Smaller than 300 μm (% by weight) | Not smaller than 150 μm Smaller than 212 μm (% by weight) | Not smaller than 45 μm Smaller than 150 μm (% by weight) | Smaller than 45 μm (% by weight) | | |
| Exaple 1 | Particulate water absorbing agent (1) | 0 | 0 | 0 | 1 | 34 | 45 | 18 | 2 | 0 | 0 | 381 | 0.22 |
| Exaple 2 | Particulate water absorbing agent (2) | 0 | 0 | 0 | 1 | 34 | 45 | 18 | 2 | 0 | 0 | 381 | 0.22 |
| Exaple 3 | Particulate water absorbing agent (3) | 0 | 0 | 0 | 1 | 34 | 49 | 15 | 1 | 0 | 0 | 386 | 0.20 |
| Comparative Exaple 1 | Comparative particulate water absorbing agent (1) | 0 | 0 | 0 | 1 | 23 | 57 | 18 | 1 | 0 | 0 | 364 | 0.20 |
| Comparative Exaple 3 | Comparative particulate water absorbing agent (3) | 0 | 0 | 0 | 1 | 23 | 57 | 18 | 1 | 0 | 0 | 381 | 0.22 |
| Comparative Exaple 4 | Comparative particulate water absorbing agent (4) | 0 | 0 | 0 | 1 | 23 | 57 | 18 | 1 | 0 | 0 | 381 | 0,22 |

EP 1 796 831 B1

24

Table 3

| | Absorbing core | Particulate water-absorbing agent used | Rewet amount for 10 min. |
|---|---|---|---|
| Example 4 | Absorbing core for evaluation (1) | Particulate water-absorbing agent (1) | 7 |
| Example 5 | Absorbing core for evaluation (2) | Particulate water-absorbing agent (2) | 8 |
| Example 6 | Absorbing core for evaluation (3) | Particulate water-absorbing agent (3) | 8 |
| Comparative Example 2 | Comparative absorbing core for evaluation (1) | Comparative particulate water-absorbing agent (1) | 9 |

A particulate water-absorbing agent of the present invention does not contain a volatile organic compound as shown in Tables 1 to 3, therefore, no odor is generated from the gel in swollen state, and the agent is superior in absorption capacity, absorbency against pressure and absorption speed. Mass median particle size and logarithmic standard deviation are also falling within a range to achieve favorable handling characteristics of the water-absorbing agent and to attain high property of the water-absorbing agent in use as an absorbing article. Use of the particulate water-absorbing agent of the present invention results in an absorbing article (a diaper in Table 3) with less rewet amount and high performance. Such a particulate water-absorbing agent of the present invention exhibits still larger blocking ratio after moisture absorption, urine resistance and deodorization performance by adding inorganic fine particles, a deodorant and the like as needed.

Industrial Applicability

As described hereinabove, the present invention relates to a particulate water-absorbing agent including a water-absorbing resin as a main component. More specifically, it relates to a particulate water-absorbing agent fulfilling more superior absorption ability without generating odor in practical applications as absorbing articles such as a diaper.

**Claims**

1.    A method for production of a particulate water-absorbing agent, which comprises:

a step of providing acrylic acid having a content of acetic acid and propionic acid of not higher than 500 ppm, and a content of acrylic acid dimer of not higher than 1000 ppm;
a reaction step of reversed phase suspension crosslinking polymerization of an unsaturated monomer containing the acrylic acid and/or a salt thereof as a main component of the monomer in a hydrocarbon solvent selected from n-hexane, n-heptane and cyclohexane;
a step of adding an aqueous solution of a surface crosslinking agent without including any organic solvent to the cross-linked polymer particles which are obtained by the reversed phase suspension crosslinking polymerization and which satisfy the following (a), (b), (c), (d) and (e):

(a) centrifuge retention capacity (CRC) in a physiological saline solution being not lower than 32 g/g;
(b) mass median particle size (D50) being not smaller than 200 $\mu$m and not larger than 400 $\mu$m;
(c) ratio of particles having diameter of smaller than 150 $\mu$m being not lower than 0 % by weight and not higher than 5 % by weight;
(d) content of volatile organic compounds as atmospheric concentration measured by a gas detector tube in accordance with the method defined in the description being not lower than 0 ppm and not higher than 100 ppm; and
(e) absorbency against pressure at 1.9 kPa in a physiological saline solution is not lower than 25 g/g; and

a step of surface crosslinking by heating at a temperature of not lower than 150° C and not higher than 250° C; and
a step of adding water insoluble inorganic fine particles, wherein the water insoluble inorganic fine particles are selected from silicon dioxide and silica acid (salt) with average particle diameter, measured by a coulter counter method, of not smaller than 0.001 and not greater than 200 $\mu$m, such that the resulting particulate water-absorbing agent has a blocking ratio after moisture absorption of not lower than 0 % by weight and not higher than 30 % by weight;

wherein the centrifuge retention capacity, the mass median particle size, the absorbency against pressure and the blocking ratio after moisture absorption are determined by the methods defined in the description; and

wherein agglomeration is carried out in the reaction step of reversed phase suspension crosslinking polymerization or

which further comprises a step of agglomeration comprising: adding water, heating in the state of keeping the water content of not lower than 1 % by weight and not higher than 10 % by weight and crushing of thus resulting aggregates.

2.  A particulate water-absorbing agent obtainable by a method for production of a particulate water-absorbing agent according to claim 1.

3.  A particulate water-absorbing agent according to claim 2, wherein said water-absorbing agent further satisfies the following (f);
    (f) content of residual monomer measured according to edana RESIDUAL MONOMERS 410.2-02 after heating at 180° C for 3 hours being not lower than 0 ppm and not higher than 500 ppm.

4.  A particulate water-absorbing agent according to claim 2 wherein logarithmic standard deviation ($\sigma\zeta$) of particle size distribution is in the range of 0.20 to 0.50 and/or ratio of particles with diameter between 150 and 600 $\mu$m based on total particles is not lower than 90 % by weight and not greater than 100 % by weight.

5.  A particulate water-absorbing agent according to claim 2 wherein amount of the water insoluble inorganic fine particles is not lower than 0.001 and not greater than 5 parts by weight based on 100 parts by weight of the water-absorbing resin in the water-absorbing agent.

6.  An absorbing article for excrement, urine or blood, which is molded by comprising a particulate water-absorbing agent according to any one of claims 2 to 5 and hydrophilic fibers.

**Patentansprüche**

1.  Verfahren zur Herstellung eines teilchenförmigen wasserabsorbierenden Mittels, das umfasst:

    einen Schritt des Bereitstellens von Acrylsäure, die einen Gehalt an Essigsäure und Propionsäure von nicht höher als 500 ppm und einen Gehalt an Acrylsäuredimer von nicht höher als 1000 ppm aufweist;
    einen Reaktionsschritt einer Umkehrphasensuspensionvernetzungspolymerisation eines ungesättigten Monomers, das die Acrylsäure und/oder ein Salz davon als einen Hauptbestandteil des Monomers in einem Kohlenwasserstofflösungsmittel, ausgewählt aus n-Hexan, n-Heptan und Cyclohexan, enthält;
    einen Schritt des Hinzugebens einer wässrigen Lösung eines Oberflächenvernetzungsmittels, ohne ein organisches Lösungsmittel einzuschließen, zu den vernetzten Polymerteilchen, welche durch die Umkehrphasensuspensionvernetzungspolymerisation erhalten werden und welche die folgenden (a), (b), (c), (d) und (e) erfüllen:

    (a) Zentrifugenretentionsvermögen (CRC) in einer physiologischen Kochsalzlösung ist nicht niedriger als 32 g/g;
    (b) Massenmedian-Teilchengröße (D50) ist nicht kleiner als 200 $\mu$m und nicht größer als 400 $\mu$m;
    (c) Anteil an Teilchen mit einem Durchmesser von kleiner als 150 $\mu$m ist nicht niedriger als 0 Gewichtsprozent und nicht höher als 5 Gewichtsprozent;
    (d) Gehalt an flüchtigen organischen Verbindungen, als atmosphärische Konzentration gemessen mit einem Gasdetektorröhrchen in Übereinstimmung mit dem in der Beschreibung definierten Verfahren, ist nicht niedriger als 0 ppm und nicht höher als 100 ppm; und
    (e) Absorptionsvermögen gegen Druck bei 1,9 kPa in einer physiologischen Kochsalzlösung ist nicht niedriger als 25 g/g; und

    einen Schritt des Oberflächenvernetzens durch Erwärmen bei einer Temperatur von nicht niedriger als 150 °C und nicht höher als 250 °C; und

einen Schritt des Hinzugebens wasserunlöslicher anorganischer Feinteilchen, wobei die wasserunlöslichen anorganischen Feinteilchen ausgewählt sind aus Siliciumdioxid und Kieselsäure(salz) mit einem mittleren Teilchendurchmesser, gemessen durch ein Coulter-Zähler Verfahren, von nicht kleiner als 0,001 und nicht größer als 200 $\mu$m, sodass das resultierende teilchenförmige wasserabsorbierende Mittel ein Blockierverhältnis nach Feuchtigkeitsabsorption von nicht niedriger als 0 Gewichtsprozent und nicht höher als 30 Gewichtsprozent aufweist;

wobei das Zentrifugenretentionsvermögen, die Massenmedian-Teilchengröße, das Absorptionsvermögen gegen Druck und das Blockierverhältnis nach Feuchtigkeitsabsorption durch die in der Beschreibung definierten Verfahren bestimmt werden; und

wobei eine Agglomeration in dem Reaktionsschritt der Umkehrphasensuspensionvernetzungspolymerisation ausgeführt wird oder

das weiter einen Agglomerationsschritt umfasst, der umfasst: Zugeben von Wasser, Erwärmen in dem Zustand, in dem der Wassergehalt von nicht weniger als 1 Gewichtsprozent und nicht höher als 10 Gewichtsprozent gehalten wird, und Zerkleinern von somit resultierenden Aggregaten.

**2.** Teilchenförmiges wasserabsorbierendes Mittel, das durch ein Verfahren zur Herstellung eines teilchenförmigen wasserabsorbierenden Mittels gemäß Anspruch 1 erhältlich ist.

**3.** Teilchenförmiges wasserabsorbierendes Mittel gemäß Anspruch 2, wobei das wasserabsorbierende Mittel weiter das folgende (f) erfüllt:

(f) Gehalt an Restmonomer, gemessen entsprechend edana RESIDUAL MONOMERS 410.2-02 nach Erwärmen bei 180 °C für 3 Stunden, ist nicht niedriger als 0 ppm und nicht höher als 500 ppm.

**4.** Teilchenförmiges wasserabsorbierendes Mittel gemäß Anspruch 2, wobei die logarithmische Standardabweichung ($\sigma\zeta$) der Teilchengrößenverteilung in dem Bereich von 0,20 bis 0,50 ist und/oder ein Anteil an Teilchen mit einem Durchmesser zwischen 150 und 600 $\mu$m basierend auf allen Teilchen nicht niedriger als 90 Gewichtsprozent und nicht größer als 100 Gewichtsprozent ist.

**5.** Teilchenförmiges wasserabsorbierendes Mittel gemäß Anspruch 2, wobei die Menge der wasserunlöslichen anorganischen Feinteilchen nicht niedriger als 0,001 und nicht größer als 5 Gewichtsteile basierend auf 100 Gewichtsteilen des wasserabsorbierenden Harzes in dem wasserabsorbierenden Mittel ist.

**6.** Absorbierender Artikel für Exkremente, Urin oder Blut, der durch das Umfassen eines teilchenförmigen wasserabsorbierenden Mittels gemäß einem der Ansprüche 2 bis 5 und hydrophiler Fasern gebildet ist.

**Revendications**

**1.** Procédé de production d'un agent particulaire absorbant l'eau, qui comprend :

une étape de fourniture d'acide acrylique ayant une teneur en acide acétique et en acide propionique pas supérieure à 500 ppm, et une teneur en dimère d'acide acrylique pas supérieure à 1000 ppm ;
une étape de réaction de polymérisation par réticulation en suspension en phase inverse d'un monomère insaturé contenant l'acide acrylique et/ou un sel de celui-ci en tant qu'un constituant principal du monomère dans un solvant hydrocarboné sélectionné parmi le n-hexane, le n-heptane et le cyclohexane ;
une étape d'ajout d'une solution aqueuse d'un agent de réticulation de surface sans inclure de solvant organique aux particules polymères réticulées qui sont obtenues par la polymérisation par réticulation en suspension en phase inverse et qui satisfont les points (a), (b), (c), (d) et (e) suivants :

(a) une capacité de rétention centrifuge (CRC) dans une solution saline physiologique n'étant pas inférieure à 32 g/g ;
(b) une taille médiane de particules en masse (D50) n'étant pas inférieure à 200 $\mu$m et pas supérieure à 400 $\mu$m ;
(c) un taux de particules ayant un diamètre inférieur à 150 $\mu$m n'étant pas inférieur à 0 % en poids et pas supérieur à 5 % en poids ;
(d) une teneur en composés organiques volatils en tant que concentration atmosphérique mesurée par un tube de détecteur de gaz conformément au procédé défini dans la description n'étant pas inférieure à 0 ppm et pas supérieure à 100 ppm ; et

(e) une capacité d'absorption contre la pression à 1,9 kPa dans une solution saline physiologique n'étant pas inférieure à 25 g/g ; et

une étape de réticulation de surface par chauffage à une température pas inférieure à 150 °C et pas supérieure à 250 °C ; et

une étape d'ajout de particules fines inorganiques insolubles dans l'eau, dans laquelle les particules fines inorganiques insolubles dans l'eau sont sélectionnées parmi le dioxyde de silicium et l'acide de silice (sel) avec un diamètre moyen des particules, mesuré par une méthode par compteur Coulter, pas inférieur à 0,001 et pas supérieur à 200 μm, de telle sorte que l'agent particulaire absorbant l'eau résultant a un taux de blocage après absorption d'humidité pas inférieur à 0 % en poids et pas supérieur à 30 % en poids ;

dans lequel la capacité de rétention centrifuge, la taille médiane des particules en masse, la capacité d'absorption contre la pression et le taux de blocage après absorption d'humidité sont déterminés par les procédés définis dans la description ; et

dans lequel l'agglomération est réalisée à l'étape de réaction de polymérisation par réticulation en suspension en phase inverse ou

qui comprend en outre une étape d'agglomération comprenant : l'ajout d'eau, le chauffage jusqu'à l'état permettant de maintenir la teneur en eau pas inférieure à 1 % en poids et pas supérieure à 10 % en poids et le broyage des agrégats en résultant.

2. Agent particulaire absorbant l'eau pouvant être obtenu par un procédé de production d'un agent particulaire absorbant l'eau selon la revendication 1.

3. Agent particulaire absorbant l'eau selon la revendication 2, dans lequel ledit agent absorbant l'eau satisfait en outre le point (f) suivant ;

(f) la teneur en monomère résiduel mesurée selon RESIDUAL MONOMERS 410.2-02 de l'Edana après chauffage à 180 °C pendant 3 heures n'étant pas inférieure à 0 ppm et pas supérieure à 500 ppm.

4. Agent particulaire absorbant l'eau selon la revendication 2 dans lequel l'écart type logarithmique ($\sigma\zeta$) de la distribution des dimensions des particules se trouve dans la plage de 0,20 à 0,50 et/ou le taux de particules ayant un diamètre compris entre 150 et 600 |jm sur la base des particules totales n'est pas inférieur à 90 % en poids et pas supérieur à 100 % en poids.

5. Agent particulaire absorbant l'eau selon la revendication 2 dans lequel la quantité de particules fines inorganiques insolubles dans l'eau n'est pas inférieure à 0,001 et pas supérieure à 5 parties en poids sur la base de 100 parties en poids de la résine absorbant l'eau dans l'agent absorbant d'eau.

6. Article absorbant pour excrément, urine ou sang, qui est moulé en comprenant un agent particulaire absorbant l'eau selon l'une quelconque des revendications 2 à 5 et des fibres hydrophiles.

FIG.1

FIG.2

FIG.3

**REFERENCES CITED IN THE DESCRIPTION**

**Patent documents cited in the description**

- US 32649 A
- GB 2267094 B
- US 5051259 A
- US 5419956 A
- US 6087002 A
- EP 0629441 A
- EP 0707603 A
- EP 0712659 A
- EP 1029886 A
- US 5462972 A
- US 5453323 A
- US 5797893 A
- US 6127454 A
- US 6184433 B
- US 6297335 B
- US 37021 A
- US 5140076 A
- US 6414214 B1
- US 5994440 A
- US 6444744 B
- US 6194531 B
- EP 0940148 A
- EP 1153656 A
- EP 0605215 A
- US 5147343 A
- US 5149335 A
- EP 0532002 A
- US 5601452 A
- US 5562646 A
- US 5669894 A
- US 6150582 A
- WO 02053198 A
- EP 0937739 A
- JP 60158861 B
- JP 11241030 B
- US 4732968 A
- WO 03095510 A
- US 6388000 B
- US 2267094 B
- EP 516925B1 A
- WO 9216565 A
- US 5744564 A
- US 4973632 A
- US 4093776 A
- US 4367323 A
- US 4446261 A
- US 4683274 A
- US 5185413 A
- US 5244735 A
- US 5998553 A
- US 6573330 B
- US 6458896 B
- US 2003153887 A
- US 6107358 A
- US 6228930 B
- US 6071976 A
- US 6254990 B
- EP 1352927 A
- US 200448955 B
- WO 200469936 A
- JP 2003280373 A
- US 4863989 A
- US 5210298 A

**Non-patent literature cited in the description**

- GENERAL CATALOGUE A-1000. SOGO LABORA-TORY GLASS WORKS CO, 2003